# EUROPÄISCHE PATENTSCHRIFT

(11) **EP 2 471 946 B1**
(45) Veröffentlichungstag und Bekanntmachung des Hinweises auf die Patenterteilung: **30.03.2016**
(21) Anmeldenummer: 11192935.2
(22) Anmeldetag: 12.12.2011
(51) Int. Cl.: C12Q 1/56, G01N 33/86

(54) **Verfahren zur Bestimmung von Inhibitoren der Gerinnung**
Method for determining coagulation inhibitors
Procédé de détermination d'inhibiteurs de coagulation

(30) Priorität: 30.12.2010 EP 10197337
(43) Veröffentlichungstag der Anmeldung: 04.07.2012
(73) Patentinhaber: Siemens Healthcare Diagnostics Products GmbH, 35041 Marburg (DE)
(72) Erfinder: Kappel, Andreas, Dr., 61462 Koenigstein (DE); Wissel, Thomas, Dr., 35094 Lahntal (DE); Rechner, Andreas, Dr., 35041 Marburg (DE); Stephan, Sina, 35043 Marburg (DE)

(56) Entgegenhaltungen:
- EP-A1- 0 034 320
- EP-A1- 0 237 332
- EP-A1- 1 918 718
- EP-A1- 2 177 624
- WO-A1-96/29347
- ULLMAN E F ET AL: "Luminescent oxygen channeling assay (LOCI (tm)): sensitive, broadly applicable homogeneous immunoassay method", CLINICAL CHEMISTRY, AMERICAN ASSOCIATION FOR CLINICAL CHEMISTRY, WASHINGTON, DC, Bd. 42, Nr. 9, 1. Januar 1996 (1996-01-01) , Seiten 1518-1526, XP002109518, ISSN: 0009-9147
- CLAESON G: "SYNTHETIC PEPTIDES AND PEPTIDOMIMETICS AS SUBSTRATES AND INHIBITORSOF THROMBIN AND OTHER PROTEASES IN THE BLOOD COAGULATION SYSTEM", BLOOD COAGULATION & FIBRINOLYSIS, RAPID COMMUNICATIONS, OXFORD, OXFORD, GB, Bd. 5, 1. Januar 1994 (1994-01-01), Seiten 411-436, XP000918308, ISSN: 0957-5235
- CAUCHON E ET AL: "Development of a homogeneous immunoassay for the detection of angiotensin I in plasma using AlphaLISA acceptor beads technology", ANALYTICAL BIOCHEMISTRY, ACADEMIC PRESS INC, NEW YORK, vol. 388, no. 1, 1 May 2009 (2009-05-01), pages 134-139, XP026081397, ISSN: 0003-2697, DOI: 10.1016/J.AB.2009.02.031 [retrieved on 2009-03-01]

## Beschreibung

Die vorliegende Erfindung liegt auf dem Gebiet der Gerinnungsdiagnostik und betrifft ein homogenes Verfahren zur Bestimmung von Inhibitoren proteolytisch aktiver Gerinnungsfaktoren (Antikoagulanzien) in einer Probe, insbesondere von direkten Thrombin- und Faktor Xa-Inhibitoren, sowie ein Testkit zur Verwendung in einem solchen Verfahren.

Gängige Antikoagulanzientherapien zielen in erster Linie auf die Hemmung der prokoagulatorischen Gerinnungsfaktoren Thrombin (Faktor IIa, FIIa) und Faktor Xa (FXa). Unterschieden wird zwischen oraler Antikoagulation mit Vitamin K-Antagonisten, wie beispielsweise Coumadin, wodurch eine Hemmung der Gerinnungsfaktorsynthese bewirkt wird, und Antikoagulation durch Hemmung der aktiven Gerinnungsfaktoren im Blutstrom. Bei den Antikoagulanzien, welche aktive Gerinnungsfaktoren im Blutstrom inhibieren bzw. inaktivieren, unterscheidet man Antikoagulanzien mit direkter und indirekter Wirkung. Antikoagulanzien mit direkter Wirkung, wie z.B. Rivaroxaban, Dabigatran oder Melagatran binden an Thrombin oder Faktor Xa sind damit hochspezifisch. Antikoagulanzien mit indirekter Wirkung, wie z.B. Heparine, binden an endogene Gerinnungsfaktorinhibitoren, wie z.B. Antithrombin, und verstärken deren antikoagulatorische Wirkung um Vielfaches.

Alle Antikoagulanzien, welche aktive Gerinnungsfaktoren im Blutstrom inhibieren, zeichnen sich durch ein spezifisches Inaktivierungsmuster aus. Bestimmte Substanzklassen, wie z.B. unfraktionierte, hochmolekulare Heparine, hemmen sowohl Thrombin als auch Faktor Xa. Andere Substanzen wirken hochspezifisch, hemmen also entweder Thrombin (z.B. Hirudin, Dabigatran, Melagatran) oder Faktor Xa (z.B. Pentasaccharide wie Fondaparinux, Rivaroxaban).

Direkte und indirekte Inhibitoren der zentralen prokoagulatorischen Gerinnungsfaktoren des Blutgerinnungssystems, Faktor Xa und Thrombin, spielen eine wachsende Rolle bei der Behandlung und Prävention kardiovaskulärer und thromboembolischer Erkrankungen. Mit den derzeit etablierten Testverfahren lassen sich diese Inhibitoren nur sehr aufwändig nachweisen. Ein einfacher und sensitiver Test auf diese Substanzen wäre sowohl für ein Therapiemonitoring als auch für den Nachweis der Anwesenheit dieser Substanzen in einer unbekannten Patientenprobe wichtig. Diese Teste müssten eine größere Anzahl strukturell nicht verwandter Thrombin- oder FXa-Inhibitoren hochsensitiv nachweisen können.

Bei den gegenwärtig angewandten chromogenen Testen zur Bestimmung von Antikoagulanzien wird die zu untersuchende Patientenprobe, die üblicherweise aus Plasma besteht, mit einem Substrat für einen Gerinnungsfaktor vermischt. Da es sich bei den meisten Blutgerinnungsfaktoren um Serin-Endopeptidasen handelt, also um Hydrolasen, die Peptidbindungen spalten können, werden vorwiegend Peptidsubstrate verwendet, die möglichst spezifisch von dem zu bestimmenden Blutgerinnungsfaktor gespalten werden und die eine nachweisbare Signalgruppe aufweisen. Bei den etablierten, auch kommerziell erhältlichen chromogenen Testen werden insbesondere die Chromophore para-Nitroanilin (pNA) und 5-Amino-2-Nitro-Benzoesäure (ANBA), welche ein Absorptionsmaximum bei 405 nm aufweisen, verwendet. Die entstehende gelbe Farbe wird in der Regel photometrisch bestimmt. Bei der Bestimmung von Antikoagulanzien verhält sich die Farbkonzentration im Testansatz umgekehrt proportional zur Antikoagulanzkonzentration in der Probe.

Bei einer ersten Gruppe von gegenwärtig angewandten chromogenen Testen zur Bestimmung von Antikoagulanzien, die die Aktivität von Blutgerinnungsfaktoren inhibieren, wird die zu untersuchende Patientenprobe üblicherweise mit einer definierten Menge eines aktivierten Gerinnungsfaktors und mit einem Substrat für diesen Gerinnungsfaktor vermischt. Je mehr Antikoagulanz in der Probe enthalten ist, desto stärker wird der aktivierte Gerinnungsfaktor inhibiert und desto weniger Substrat wird gespalten. Kommerziell erhältliche Teste, die auf diesem Testprinzip beruhen, sind beispielsweise der Berichrom® Heparin Test von Siemens Healthcare Diagnostics zur Bestimmung von Heparin anhand der Hemmung von zugegebenem Faktor Xa oder der Hirudin Activity Assay von Siemens Healthcare Diagnostics zur Bestimmung von Hirudin anhand der Hemmung von zugegebenem Thrombin.

Bei einer zweiten Gruppe von gegenwärtig angewandten chromogenen Testen zur Bestimmung von Antikoagulanzien wird die zu untersuchende Patientenprobe mit einem inaktiven Gerinnungsfaktor, einem Gerinnungsaktivator und mit einem chromogenen Substrat für den Gerinnungsfaktor vermischt. Durch die Zugabe des Gerinnungsaktivators wird der zugegebene inaktive Gerinnungsfaktor zunächst aktiviert. Je mehr Antikoagulanz in der Probe enthalten ist, desto stärker wird der aktivierte Gerinnungsfaktor inhibiert und desto weniger Substrat wird gespalten. Kommerziell erhältliche Teste, die auf diesem Testprinzip beruhen, sind beispielsweise der Haemosys®-ECA T Test von JenAffin zur Bestimmung von synthetischen direkten Thrombininhibitoren oder der Haemosys®-ECA H Test von JenAffin zur Bestimmung von Hirudin anhand der Hemmung von Thrombin/Meizothrombin, welches durch die Zugabe von Prothrombin und Ecarin als Gerinnungsaktivator in der Probe gebildet wird.

Ein weiteres Testprinzip zur Bestimmung von Antikoagulanzien ist in EP-A1-2 177 625 beschrieben. Bei diesem Testprinzip wird ebenfalls die Spaltung eines Gerinnungsfaktorspezifischen Substrats bestimmt, allerdings nicht mit Hilfe chromogener Substrate, sondern mit Hilfe eines andersartigen signalbildenen Systems. Dieses System umfasst zwei Komponenten, die, wenn sie gleichzeitig an das intakte Substrat binden durch die räumliche Nähe wechselwirken und ein detektierbares Signal generieren, beispielsweise Fluoreszenz oder Chemilumineszenz. Infolge der Spaltung einer Peptidbindung des Substrats werden die beiden Komponenten voneinander getrennt, wodurch eine Signalbildung unterbleibt. Je mehr Antikoagulanz in der Probe enthalten ist, desto stärker wird der aktivierte Gerinnungsfaktor inhibiert, desto weniger Substrat wird gespalten und desto stärker ist die Signalbildung. Der Vorteil Fluoreszenz- oder Chemilumineszenz-basierter Testverfahren gegenüber den chromogenen Testen besteht grundsätzlich darin, dass sie sensitiver sind und außerdem Messungen in Vollblut erlauben.

Der Nachteil des vorbeschriebenen Verfahrens, bei dem ein spaltbares Substrat gewissermaßen zwischen den beiden Komponenten des signalgebenden Systems "aufgespannt" wird, besteht darin, dass der Substratligand beiderseits der Spaltstelle Bereiche für die Assoziation mit den signalgebenden Komponenten aufweisen muss. Üblicherweise handelt es sich bei den Substratliganden um synthetische, niedermolekulare Peptidsubstrate, die für die Assoziation mit den signalgebenden Komponenten zwei artifizielle Reste aufweisen, wie beispielsweise ein aminoterminales Flag-Tag und einen carboxyterminalen Biotin-Rest. Die Kopplung derartiger Reste an niedermolekulare Peptidsubstrate ist jedoch immer mit der Gefahr verbunden, dass die Struktur des Peptids so verändert wird, dass es von dem nachzuweisenden Enzym nicht mehr gebunden oder nicht gespalten wird, wodurch die Bereitstellung geeigneter Substratliganden technisch aufwändig ist.

Es ist also wünschenswert, bekannte Verfahren zur Bestimmung von Antikoagulanzien, bei denen zwei Komponenten verwendet werden, die durch räumliche Nähe wechselwirken und ein detektierbares Signal generieren, so zu modifizieren, dass niedermolekulare Liganden verwendet werden können, die maximal einen artifiziellen Rest für die Assoziation mit einer der signalgebenden Komponenten aufweisen müssen.

Diese Aufgabe wird dadurch gelöst, dass ein Aliquot einer Probe, die vermutlich ein Antikoagulanz enthält, mit einer definierten Menge eines proteolytisch aktiven Gerinnungsfaktors und mit einem Liganden, welcher an das aktive Zentrum des proteolytisch aktiven Gerinnungsfaktors bindet, aber nicht von diesem an einer Peptidbindung gespalten wird, vermischt wird, und das Signal, das von dem signalbildenden System infolge der Bindung des Liganden an den aktivierten Gerinnungsfaktor generiert wird, gemessen wird. Der in der Probe enthaltene Inhibitor des aktivierten Gerinnungsfaktors, d.h. das Antikoagulanz, konkurriert konzentrationsabhängig mit dem Liganden um die Bindung an das aktive Zentrum des proteolytisch aktiven Gerinnungsfaktors und hemmt somit die Signalgenerierung. Je mehr Antikoagulanz in der Probe enthalten ist, desto weniger Signal wird generiert.

Die vorliegende Erfindung betrifft daher ein Verfahren zur Bestimmung eines Inhibitors eines proteolytisch aktiven Gerinnungsfaktors, d.h. eines Antikoagulanzes, in einer Probe, umfassend die Schritte:
a) Bereitstellen und Inkubieren eines Reaktionsansatzes, enthaltend
   i. ein Aliquot der Probe,
   ii. eine definierte Menge eines proteolytisch aktiven Gerinnungsfaktors,
   iii. mindestens einen Liganden, welcher an das aktive Zentrum des proteolytisch aktiven Gerinnungsfaktors bindet, aber nicht von diesem an einer Peptidbindung gespalten wird,
   iv. eine erste und eine zweite Komponente eines signalbildenden Systems, welche so zusammenwirken, dass ein detektierbares Signal entsteht, wenn die erste und die zweite Komponente des signalbildenden Systems in räumliche Nähe zueinander gebracht
   werden und wobei der proteolytisch aktive Gerinnungsfaktor mit der ersten Komponente des signalbildenden Systems assoziiert ist oder während der Inkubation assoziiert wird und wobei der Ligand mit der zweiten Komponente des signalbildenden Systems assoziiert ist oder während der Inkubation assoziiert wird;
b) Messen des Signals des signalbildenden Systems, wobei sich die Signalhöhe umgekehrt proportional zur Antikoagulanzkonzentration in der Probe verhält.

Der Begriff "Antikoagulanz" wird in Bezug auf die vorliegende Erfindung für Inhibitoren proteolytisch aktiver Gerinnungsfaktoren verwendet, die natürlichen oder synthetischen Ursprungs sind und deren beabsichtigte Verwendung die Hemmung des Gerinnungssystem *in vivo* in Mensch oder Tier ist. Dazu gehören auch hochmolekulare Substanzen wie Hirudin oder Heparin, die ein Enzym an mehren Bindungsstellen binden, oder aber auch niedermolekulare Verbindungen, wie Rivaroxaban, Dabigatran oder Melagatran, für die es hinreichend ist, nur am aktiven Zentrum des Enzyms zu binden, um die Reaktivität zu inhibieren. Die Begriffe "Antikoagulanz" und "Inhibitor eines proteolytisch aktiven Gerinnungsfaktors" werden im Sinne der Erfindung synonym verwendet.

Unter dem Begriff "Ligand" sind in Bezug auf die vorliegende Erfindung Substanzen zu verstehen, die im aktiven Zentrum eines proteolytisch aktiven Gerinnungsfaktors binden und daher mit einem Inhibitor um die Bindungsstelle konkurrieren. Geeignete Liganden werden jedoch nicht von dem proteolytisch aktiven Gerinnungsfaktor an einer Peptidbindung gespalten, weil ihnen eine durch den proteolytisch aktiven Gerinnungsfaktor spaltbare Peptidbindung fehlt, d.h. sie weisen keine durch den proteolytisch aktiven Gerinnungsfaktor hydrolysierbare Peptidylbindung (R-CONH-R, R = Aminosäurerest) auf. Es sind sowohl reversibel als auch irreversibel bindende Liganden geeignet.

Der Ligand kann ein synthetisch, rekombinant oder biotechnologisch hergestelltes Molekül oder ein natürlicherweise vorkommendes Molekül sein.

Ein Ligand kann ein Peptidderivat sein, dessen Sequenz von der Sequenz eines natürlichen Substrats des Enzyms abgeleitet ist. Ein Peptidderivat-Ligand im Sinne der vorliegenden Erfindung unterscheidet sich von einem Peptidsubstrat dadurch, dass er nicht durch den proteolytisch aktiven Gerinnungsfaktor hydrolytisch gespalten wird, während bei der Spaltung eines Peptidsubstrats Fragmente entstehen, die aus dem aktiven Zentrum des Enzyms freigesetzt werden, und das Enzym unverändert aus der Reaktion wieder hervorgeht.

Bevorzugterweise umfassen geeignete Peptidderivate 3 bis etwa 150 Aminosäurereste. Um nicht von dem proteolytisch aktiven Gerinnungsfaktor an einer Peptidbindung gespalten werden zu können, ist die carboxyterminale Carboxylgruppe eines erfindungsgemäßen Peptidderivats bevorzugterweise durch eine funktionelle Gruppe aus der Gruppe Aldehyd (-CHO), Keton (-COR; R=Alkyl oder Aryl), Trifluormethylketon (-COCF₃), α-Ketokarbonsäure (-COCOOH), α-Ketoamid (-COCONHR; R=Alkyl oder Aryl) und α-Ketoester (-COCOOR; R=Alkyl oder Aryl), Ester (-COOR; R=Alkyl oder Aryl), Boronsäure (-B(OR)₂), Chlormethylketone (-COCH₂Cl) und Sulfonylfluoride (-SO₂F) ersetzt. Das Peptidderivat kann weitere strukturelle Änderungen aufweisen, z.B. die teilweise Verwendung von D-Aminosäuren anstelle von natürlich vorkommenden L-Aminosäuren.

Tabelle 1 enthält Beispiele für typische funktionelle Gruppen für reversibel und irreversibel bindende Peptidderivat-Liganden.

**Tabelle 1**

| **Ligandentyp** | **Funktionelle Gruppe** | |
|---|---|---|
| irreversibel | Chlormethylketon | -COCH₂Cl |
| | Sulfonylfluorid | -SO₂F |
| | Ester | -COOR |
| | | (R=Alkyl oder Aryl) |
| | Boronsäure | B(OR₂) |
| | | (R=Alkyl oder Aryl) |
| reversibel | Aldehyd | -CHO |
| | Keton | -COR |
| | | (R=Alkyl oder Aryl) |
| | Trifluormethylketon | -COCF₃ |
| | α-Ketokarbonsäure | -COCOOH |
| | α-Ketoamid | -COCONHR |
| | | (R=Alkyl oder Aryl) |
| | α-Ketoester | -COCOOR |
| | | (R=Alkyl oder Aryl) |

Beispiele für Peptidderivat-Liganden, die im aktiven Zentrum von Thrombin binden, von diesem aber nicht an einer Peptidbindung gespalten werden, sind:
- H-D-Phe-Pro-Arg-H
- Me-D-Phe-Pro-Arg-H
- H-D-Phe-Pro-Agm
- H-D-Phe-Pro-Arg-CH₂-Cl
- H-D-Phe-Pro-Arg-CH₂F
- Boc-DL-Dpa-Pro-Arg-H
- Ac-D-Phe-Pro-boroArg pinanediol Ester
- D-Phe-Pro-NH-CH(Methoxypropyl)-P(O)(OPh)₂

Beispiele für Peptidderivat-Liganden, die im aktiven Zentrum von Faktor Xa binden, von diesem aber nicht an einer Peptidbindung gespalten werden, sind:
- D-Arg-Gly-Arg-H
- Dansyl-Glu-Gly-Arg-CH₂Cl

Bezüglich der genannten Peptidderivat-Liganden siehe auch Claeson, G., Blood Coagulation and Fibrinolysis 5, 1994, 411-436.

Ein Ligand kann ferner eine Verbindung sein, die nicht zur Gruppe der Peptidverbindungen gehört, wie beispielsweise ein Arginin-, ein Amidinophenylalanin-, ein p-Guanidinophenylalanin-, ein 3-Amidinophenylalaninester, ein dibasisches (Amidinoaryl)propanoiksäure- oder ein Oxazolidinonderivat.

Argininderivat-Liganden, die im aktiven Zentrum von Thrombin binden, von diesem aber nicht an einer Peptidbindung gespalten werden, sind z.B. Nα-arysulfonylargininamide (R₁₋SO₂-Arg-N-R₂/R₃; R₁=hydrophobe aliphatische oder aromatische Gruppe) (Claeson, G., Blood Coagulation and Fibrinolysis 5, 1994, 411-436).

Amidinophenylalaninderivat-Liganden, die im aktiven Zentrum von Thrombin binden, von diesem aber nicht an einer Peptidbindung gespalten werden, sind z.B. Nα-(ß-naphthylsulphonyl)-4-amidinophenylalanine piperidid oder Nα-(ß-naphthylsulphony-glyclyl)-4-amidinophenylalanine piperidid (Claeson, G., Blood Coagulation and Fibrinolysis 5, 1994, 411-436).

Ein p-Guanidinophenylalaninderivat-Ligand, der im aktiven Zentrum von Thrombin bindet, von diesem aber nicht an einer Peptidbindung gespalten wird, ist z.B. Tos-Gpa-piperidid (Claeson, G., Blood Coagulation and Fibrinolysis 5, 1994, 411-436).

Ein 3-Amidinophenylalaninesterderivat-Ligand, der im aktiven Zentrum von Faktor Xa bindet, von diesem aber nicht an einer Peptidbindung gespalten wird, ist z.B. Nα-(ß-naphtylsulphonyl-glycyl)-3-amidinophenylalanin (Claeson, G., Blood Coagulation and Fibrinolysis 5, 1994, 411-436).

Ein dibasischer (Amidinoaryl)propanoiksäurederivat-Ligand, der im aktiven Zentrum von Faktor Xa bindet, von diesem aber nicht an einer Peptidbindung gespalten wird, ist z.B (2S)-2-[4[[(3S)-1-acetimidoyl-3-pyrrolidinyl]oxy]phenyl]-3-(7-amidino-2-naphtyl)propanoiksäure (Nagahara, T. et al., J. Med. Chem. 1994, 37, 1200-1207).

Ein Oxazolidinonderivat-Ligand, der im aktiven Zentrum von Faktor Xa bindet, von diesem aber nicht an einer Peptidbindung gespalten wird, ist z.B. 5-chloro-N-({(5S)-2-oxo-3-[4-(3-oxomorpholin-4-yl)phenyl]-1,3-oxazolidin-5-yl}methyl)thiophene-2-carboxamid (Roehrig, S., et al., J. Med. Chem. 2005, 48(19), 5900-5908).

Unter einem reversibel bindenden Liganden versteht man einen Liganden, der konzentrationsabhängig von mindestens einem natürlichen oder synthetischen Inhibitor aus dem aktiven Zentrum des proteolytisch aktiven Gerinnungsfaktors verdrängt werden kann.

Beispiele für geeignete reversibel an Thrombin bindende Liganden sind Peptidderivate, wie z.B H-D-Phe-Pro-Arg-H, Me-D-Phe-Pro-Arg-H oder H-D-Phe-Pro-Agm. In Agmantin (Agm)ist die Carboxlgruppe durch Wasserstoff ersetzt. Andere reversibel an Thrombin bindende Liganden sind Nα-arysulfonylargininamid (R₁-SO₂-Arg-N-R₂/R₃) oder Amidinopiperidinderivate wie Nα-(ß-naphthylsulphonyl)-4amidinophenylalanine piperidid oder Derivate von p-Guanidinophenylalanin (Gpa), amidinophenylalanin (Apa) wie z.B. Tos-Gpa-piperidid und Tos-Apa-piperidid oder auch Peptidaminoboronsäuren oder Peptidaminophosphonsäuren wie z.B. Ac-D-Phe-Pro-NH-CH(CH₂-Phenyl)-B-OPin und D-Ala-Pro-NH-CH(Methoxypropyl)-P(O)(OPh)₂.

Beispiele für geeignete reversibel an Faktor Xa bindende Liganden sind Peptidderivate wie z.B. D-Arg-Gly-Arg-H oder Benzamidinderivate und 3-Amidinophenylalaninester wie z.B. Nα-(ß-naphtylsulphonyl -glycyl)-3-amidinophenylalanin.

Unter einem irreversibel bindenden Liganden versteht man einen Liganden, der konzentrationsunabhängig an das aktive Zentrum des proteolytisch aktiven Gerinnungsfaktors bindet und der nicht mehr verdrängt werden kann, weil er z.B. eine kovalente Bindung mit dem Enzym eingeht und das Enzym in einer Weise verändert, dass das aktive Zentrum irreversibel inaktiviert ist. Typische irreversibel bindende Liganden bestehen aus einem Teil, der sie in das aktive Zentrum des Enzyms einführt und einer chemisch reaktiven Gruppe, die in der Lage ist, kovalente Bindungen zum Enzym, d.h. im Falle der proteolytisch aktiven Gerinnungsfaktoren zur Hydroylfunktion des Serins im aktiven Zentrum auzubilden. Zur Ausbildung der kovalenten Bindung mit dem Enzym wird ein Teil der funktionellen Gruppe aus dem Liganden abgespalten, im gleichen Reaktionschritt bindet der Ligand dann kovalent an das Enzym.

Beispiele für geeignete irreversibel an Thrombin bindende Liganden sind H-D-Phe-Pro-Arg-CH₂-Cl oder H-D-Phe-Pro-Arg-CH₂F.

Ein Beispiel für einen geeigneten irreversibel an Faktor Xa bindenden Liganden ist Dansyl-Glu-Gly-Arg-CH₂Cl.

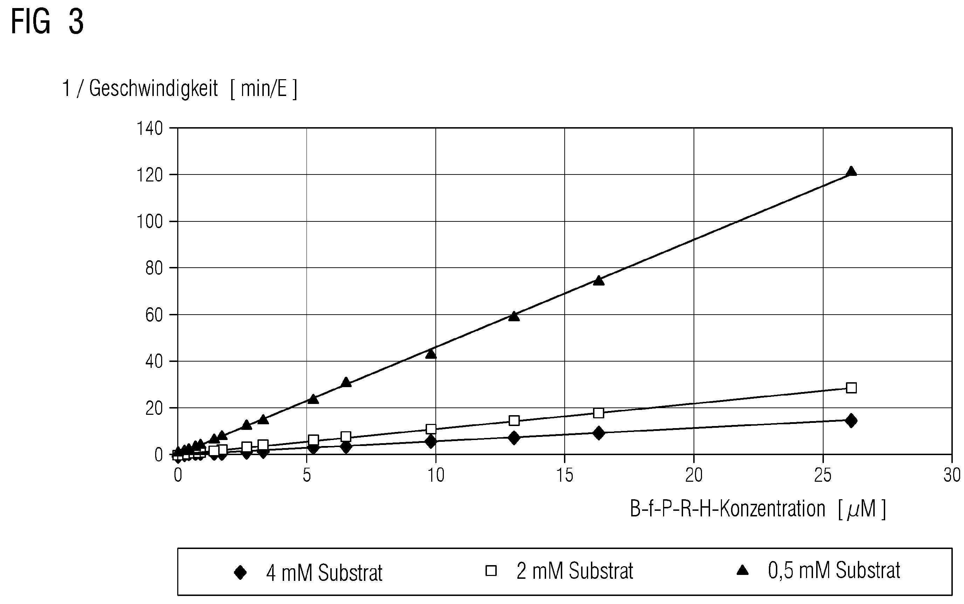

Reversibel und irreversibel bindende Liganden können durch ihre Reaktionskinetik unterschieden werden. Trägt man 1/v, wobei v die Reaktionsgeschwindigkeit ist, gegen die Ligandenkonzentration (i) bei konstanter Substratkonzentration (S) auf, so erhält man eine Gerade. Bei Verwendung von zwei oder mehreren unterschiedlichen Substratkonzentrationen (S₁, S₂... S_{N}) schneiden sich die Geraden in einem Punkt. Der Wert der Abszissenachse dieses Punktes entspricht -Kᵢ, wobei Kᵢ der Bindungskonstante entspricht, siehe Figur 3 Ausschnitt (Dixon, M., 1953, Biochem J, 55, 170-171). Bei einer irreversiblen Hemmung treffen sich die Kurven an einem Punkt auf der Abszisse, der widerum -Kᵢ entspricht.

Bevorzugte Liganden haben eine Bindungsaffinität zu dem proteolytisch aktiven Gerinnungsfaktor, die der Affinität des zu bestimmenden Antikoagulanzes entspricht oder niedriger ist. Als Antikoagulanzien verwendete Thrombininhibitoren zeigen eine Bindungskonstante für Thrombin im Bereich von 2,7 × 10⁻¹⁴ Mol (Hirudin) bis 1,9 × 10⁻⁸ Mol (Argatroban) (Prasa, D. et al., Thromb Haemost 77, 1997, 498-503). Die Thrombininhibitoren binden neben Thrombin auch F Xa, wenn auch mit wesentlich geringerer Affinität, d.h. mit einer größeren Bindungkonstante von 2,1 × 10⁻⁴ Mol, wie z.B. Argatroban (Prasa, D. et al., Thromb Haemost 78, 1997, 1215-1220). F Xa-Inhibitoren haben eine Bindungskonstante im Bereich von 8 × 10⁻¹¹ Mol (Apixaban) bis 6,6 × 10⁻⁹ Mol (LY517717 difumarate), wobei auch die F Xa-Inhibitoren in gewissem Umfang an Thrombin binden, allerdings um bis zu 10.000fach schwächer, wie z.B. für Rivaroxaban (Perzborn, E., Hämostaseologie 3, 2009, 260-267). Reversible Liganden können somit von dem zu bestimmenden Inhibitor vom aktiven Zentrum des proteolytisch aktiven Gerinnungsfaktors verdrängt werden. Irreversible Liganden mit einer entsprechend ausgewählten Affinität, können somit das zu bestimmende Medikament nicht in einem relevanten Zeitraum vom aktiven Zentrum des proteolytisch aktiven Gerinnungsfaktors verdrängen. Andererseits darf die Affinität nicht zu niedrig sein, da sonst kein Signal entsteht kann.

Die Bindungskonstante geeigneter Liganden liegt daher bevorzugterweise in einem Bereich von 0,0001 bis 100.000 nM (10⁻¹³ bis 10⁻⁴ M), besonders bevorzugt in einem Bereich von 0,1 bis 10.000 nM (10⁻¹⁰ bis 10⁻⁵ M), ganz besonders bevorzugt in einem Bereich von 1 bis 1000 nM (10⁻⁹ bis 10⁻⁶ M) .

Unter dem Begriff "Probe" ist im Sinne der vorliegenden Erfindung das Material zu verstehen, das das nachzuweisende Antikoagulanz vermutlich enthält. Der Begriff "Probe" umfasst insbesondere biologische Flüssigkeiten oder Gewebe von Menschen und Tieren wie Blut, Plasma, Serum sowie andere Körperflüssigkeiten, Ausscheidungen oder Extrakte.

Das erfindungsgemäße Verfahren umfasst die Bereitstellung und Inkubation eines Reaktionsansatzes enthaltend ein Aliquot der Probe, eine definierte Menge eines proteolytisch aktiven Gerinnungsfaktors, mindestens einen Liganden, welcher an das aktive Zentrum des proteolytisch aktiven Gerinnungsfaktors bindet, aber nicht von diesem an einer Peptidbindung gespalten wird, eine erste und eine zweite Komponente eines signalbildenden Systems, welche so zusammenwirken, dass ein detektierbares Signal entsteht, wenn die erste und die zweite Komponente des signalbildenden Systems in räumliche Nähe zueinander gebracht werden. Der proteolytisch aktive Gerinnungsfaktor ist oder wird während der Inkubation mit der ersten Komponente des signalbildenden Systems assoziiert. Der Ligand ist oder wird während der Inkubation mit der zweiten Komponente des signalbildenden Systems assoziiert. Infolge der Bindung des Liganden an das aktive Zentrum des proteolytisch aktiven Gerinnungsfaktors werden die Komponenten des signalbildenden Systems in räumliche Nähe zueinander gebracht, wodurch ein detektierbares Signal entsteht, welches mit der Aktivität des eines proteolytisch aktiven Gerinnungsfaktors im Reaktionsansatz korreliert. Das in der Probe enthaltene Antikoagulanz konkurriert konzentrationsabhängig mit dem nicht an einer Peptidbindung spaltbaren Liganden um die Bindung an das aktive Zentrum des proteolytisch aktiven Gerinnungsfaktors und hemmt somit die Signalgenerierung. Je mehr Antikoagulanz in der Probe enthalten ist, desto weniger Signal wird generiert. Das gebildete Signal wird gemessen, wobei sich die Signalhöhe umgekehrt proportional zur Antikoagulanzkonzentration in der Probe verhält.

Das erfindungsgemäße Verfahren eignet sich zur Bestimmung von Inhibitoren der proteolytisch aktiven Gerinnungsfaktoren Thrombin (Faktor IIa), Faktor VIIa, Faktor IXa, Faktor Xa, Faktor XIa oder Faktor XIIa.

Von der Spezifität des zu bestimmenden Antikoagulanzes hängt es ab, welcher aktivierte Gerinnungsfaktor und welcher Ligand dem Reaktionsansatz hinzugefügt wird.

Für die Bestimmung eines Heparins, d.h. eines hochmolekularen, unfraktionierten Heparins (HMW Heparin) oder eines niedermolekularen Heparins (LMW Heparin) oder eines Heparinoids, eignet sich insbesondere die Zugabe von Thrombin oder von Faktor Xa und die Zugabe eines Liganden mit Thrombin- oder Faktor Xa-Spezifität. Für die Bestimmung eines direkten Thrombininhibitors, z.B. von Argatroban, Melagatran, Ximelagatran, Bivalirudin, Dabigatran, Hirudin, Lepirudin, MCC-977, SSR-182289, TGN-255, TGN-167, ARC-183 und Odiparcil, eignet sich insbesondere die Zugabe von Faktor IIa (Thrombin) und die Zugabe eines Liganden mit Thrombinspezifität. Für die Bestimmung eines direkten Faktor Xa-Inhibitors, z.B. von Rivaroxaban; Apixaban; Otamixaban, welche in der neuen Wikstoffklasse der Xabane zusammengefasst werden; Fondaparinux; LY 517717; YM 153; DU-176b; DX-9065a und KFA-1982 eignet sich insbesondere die Zugabe von Faktor Xa und eines Faktor Xa-spezifischen Liganden.

Bei dem dem Reaktionsansatz hinzugefügten proteolytisch aktiven Gerinnungsfaktor kann es sich um einen Gerinnungsfaktor handeln, der aus tierischem oder humanem Gewebe oder Körperflüssigkeiten, wie z.B. Blut oder Plasma, isoliert wurde oder der aus Überständen oder Lysaten tierischer oder humaner Zellkulturen, oder Kulturen eukaryontischer Zellen oder von Mikroorganismen, wie Bakterien oder Pilzen, die einen rekombinanten Gerinnungsfaktor exprimieren, isoliert wurde. Verfahren zur Aktivierung von zunächst inaktiven Zymogenen der verschiedenen Gerinnungsfaktoren sind dem Fachmann hinlänglich bekannt. Welcher aktivierte Gerinnungsfaktor dem Reaktionsansatz zugegeben wird, hängt davon ab, welches Antikoagulanz bestimmt werden soll.

In einer Ausführungsform des erfindungsgemäßen Verfahrens weist der proteolytisch aktive Gerinnungsfaktor einen ersten Bindungspartner X eines Bindungspaares X/Y auf und die erste Komponente des signalbildenden Systems ist mit dem zweiten Bindungspartner Y des Bindungspaares X/Y assoziiert, wodurch der proteolytisch aktive Gerinnungsfaktor durch Bindung der Bindungspartner X und Y an die erste Komponente des signalbildenden Systems gebunden ist oder während der Inkubation des Reaktionsgemisches an die erste Komponente des signalbildenden Systems gebunden wird.

Die Bindungspartner X und Y sind zwei unterschiedliche Moleküle, die einander spezifisch erkennen und binden. Beispiele für spezifische Erkennung und Bindung sind Antikörper-Antigen-Wechselwirkungen, Polynukleotid-Wechselwirkungen etc.

Geeignete Bindungspaare X/Y sind vor allem Antigen/Antikörper-Kombinationen, wobei der Bindungspartner X ein antigenes Epitop des proteolytisch aktiven Gerinnungsfaktors ist. Das antigene Epitop kann ein natürliches Sequenz- oder Strukturepitop des natürlichen Proteins sein. Das antigene Epitop kann auch ein heterologes Sequenz- oder Strukturepitop eines modifizierten aktiven Gerinnungsfaktors sein. Beispiele für heterologe Sequenz- oder Strukturepitope sind FLAG-, oder HIS- oder Fluorescein-Tags, die insbesondere zur Markierung von Peptiden oder Proteinen verwendet werden. Weitere geeignete Bindungspaare X/Y sind komplementäre Polynukleotide X und Y. Der mit der ersten Komponente des signalbildenden Systems assoziierte Bindungspartner Y ist so zu wählen, dass proteolytisch aktive Gerinnungsfaktor spezifisch gebunden werden kann. Bevorzugterweise besteht der Bindungspartner Y aus einem Antikörper oder einem Antigen-bindenden Fragment desselben. Besonders bevorzugte Bindungspaare X/Y sind FLAG-Tag/anti-FLAG-Tag-Antikörper, HIS-Tag/anti-HIS-Tag-Antikörper Fluorescein/anti-Fluorescein-Antikörper, Biotin/Avidin und Biotin/Streptavidin.

In einer anderen Ausführungsform des erfindungsgemäßen Verfahrens ist der proteolytisch aktive Gerinnungsfaktor über eine kovalente Bindung mit der ersten Komponente des signalbildenden Systems verbunden.

In einer bevorzugten Ausführungsform weist der Ligand, der dem Reaktionsansatz hinzugefügt wird, einen ersten Bindungspartner A eines ersten Bindungspaares A/B auf zur Bindung an die zweite Komponente des signalbildenden Systems, welche passenderweise den zweiten Bindungspartner B des Bindungspaares A/B aufweist. Besonders bevorzugterweise weist der Ligand mindestens einen Biotinrest als Bindungspartner A auf.

Die Bindungspartner A und B sind zwei unterschiedliche Moleküle, die einander spezifisch erkennen und binden. Beispiele für spezifische Erkennung und Bindung sind Antikörper-Antigen-Wechselwirkungen, Polynukleotid-Wechselwirkungen etc.

Geeignete Bindungspaare A/B sind vor allem Antigen/ Antikörper-Kombinationen, wobei der Bindungspartner A ein antigenes Epitop des Liganden ist. Das antigene Epitop kann ein natürliches Sequenz- oder Strukturepitop eines natürlichen Proteins oder Proteinfragmentes sein. Das antigene Epitop kann auch ein heterologes Sequenz- oder Strukturepitop eines modifizierten Peptids sein. Beispiele für heterologe Sequenz- oder Strukturepitope sind FLAG-, oder HIS- oder Fluorescein-Tags, die insbesondere zur Markierung von niedermolekularen Substanzen, Peptiden oder Proteinen verwendet werden. Weitere geeignete Bindungspaare A/B sind komplementäre Polynukleotide A und B. Der mit der zweiten Komponente des signalbildenden Systems assoziierte Bindungspartner B ist so zu wählen, dass der Ligand spezifisch gebunden werden kann. Bevorzugterweise besteht der Bindungspartner B aus einem Antikörper oder einem Antigen-bindenden Fragment desselben. Besonders bevorzugte Bindungspaare A/B sind FLAG-Tag/anti-FLAG-Tag-Antikörper, HIS-Tag/anti-HIS-Tag-Antikörper, Fluorescein/anti-Fluorescein-Antikörper, Biotin/Avidin und Biotin/Streptavidin.

Ein etwaiges Bindungspaar A/B zur Assoziation des Liganden mit der zweiten signalgebenden Komponente ist grundsätzlich so zu wählen, dass es mit einem etwaigen Bindungspaar X/Y zur Assoziation des proteolytisch aktiven Gerinnungsfaktors mit der ersten signalgebenden Komponente nicht durch gegenseitige Bindung interferiert.

Ein Vorteil der vorliegenden Erfindung besteht darin, dass der verwendete Ligand einen zweiten artifiziellen Bindungspartner eines Bindungspaares zwar aufweisen kann, aber nicht aufweisen muss.

Unter dem Begriff "signalbildendes System" ist im Sinne der vorliegenden Erfindung ein System zu verstehen, das mindestens eine erste und eine zweite Komponente umfasst, welche so zusammenwirken, dass ein detektierbares Signal entsteht, wenn sie in räumliche Nähe zueinander gebracht werden.

In einer Ausführungsform handelt es sich bei den Komponenten des signalbildenden Systems um partikuläre Festphasen, beispielsweise Latexpartikel, deren Agglutination turbidimetrisch oder nephelometrisch bestimmt wird. Dazu besteht die erste Komponente des signalbildenden Systems aus einer ersten partikulären Festphase, die so beschaffen ist, dass sie mit dem proteolytisch aktiven Gerinnungsfaktor assoziiert ist oder assoziierbar ist. Die erste partikuläre Festphase kann mit dem proteolytisch aktiven Gerinnungsfaktor über eine kovalente Bindung oder über ein Bindungspaar X/Y verbunden sein oder über ein Bindungspaar X/Y im Reaktionsansatz verbunden werden. Weiterhin besteht die zweite Komponente des signalbildenden Systems aus einer zweiten partikulären Festphase, die so beschaffen ist, dass sie mit dem Liganden assoziiert ist oder assoziierbar ist. Die zweite partikuläre Festphase kann mit dem Liganden über eine kovalente Bindung oder über ein Bindungspaar A/B verbunden sein oder über ein Bindungspaar A/B im Reaktionsansatz verbunden werden. Immunoassays beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden Polystyrolpartikel mit einem Durchmesser von 0,1 bis 0,5 µm, besonders bevorzugt mit einem Durchmesser von 0,15 0,35 µm verwendet. Bevorzugt werden Polystyrolpartikel mit Carboxyl- oder Aldehydfunktionen verwendet. Weiterhin bevorzugt werden Schale/Kern-Partikel verwendet. Die Synthese der Partikel und die kovalente Kopplung von Liganden ist z.B. in Peula, J.M. et al., Covalent coupling of antibodies to aldehyde groups on polymer carriers. Journal of Materials Science: Materials in Medicine 1995; 6: 779-785 beschrieben.

In einer anderen Ausführungsform des erfindungsgemäßen Verfahrens umfasst das signalbildende System mindestens eine erste und eine zweite Komponente, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z. B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI® Technologie) oder Photosensitizer und Fluorophor (WO 95/06877) oder radioaktives Iod<125> und Fluorophor (Udenfriend et al. (1985) Proc. Natl. Acad. Sci. 82: 8672 - 8676) oder Fluorophor und Fluoreszenz-Quencher (US 3,996,345).

Die erste Komponente und/oder die zweite Komponente des signalbildenden Systems, die miteinander in Wechselwirkung treten können, können mit einer partikulären Festphase kovalent oder über spezifische Wechselwirkung assoziiert oder in diese eingelagert sein. Unter dem Begriff partikuläre Festphase sind suspendierbare Teilchen zu verstehen, wie z.B. Metallsolen, Silica-Partikel, Magnetpartikel oder besonders bevorzugt Latexpartikel. Bevorzugt werden Partikel mit einem Durchmesser von 0,01 - 10 Mikrometer, insbesondere bevorzugt werden Partikel mit einem Durchmesser von 0,1 - 1 Mikrometer.

Die erste Komponente des signalbildenden Systems, dessen Komponenten so zusammenwirken, dass ein detektierbares Signal entsteht, wenn sie in räumliche Nähe zueinander gebracht werden und dadurch miteinander in Wechselwirkung treten können, ist so beschaffen, dass sie mit dem proteolytisch aktiven Gerinnungsfaktor assoziiert ist oder assoziierbar ist. Die erste Komponente des signalbildenden Systems kann direkt mit dem proteolytisch aktiven Gerinnungsfaktor assoziiert sein oder assoziierbar sein. Bevorzugterweise ist die erste Komponente des signalbildenden Systems indirekt mit dem proteolytisch aktiven Gerinnungsfaktor assoziiert oder assoziierbar. Dazu ist die erste Komponente des signalbildenden Systems mit einer partikulären Festphase assoziiert, welche zusätzlich mit dem proteolytisch aktiven Gerinnungsfaktor kovalent oder über ein Bindungspaar X/Y assoziiert ist oder über ein Bindungspaar X/Y assoziierbar ist.

Die zweite Komponente des signalbildenden Systems, dessen Komponenten so zusammenwirken, dass ein detektierbares Signal entsteht, wenn sie in räumliche Nähe zueinander gebracht werden und dadurch miteinander in Wechselwirkung treten können, ist so beschaffen, dass sie mit dem Liganden assoziiert ist oder assoziierbar ist. Die zweite Komponente des signalbildenden Systems kann direkt mit dem Liganden assoziiert sein oder assoziierbar sein. Bevorzugterweise ist die zweite Komponente des signalbildenden Systems indirekt mit dem Liganden assoziiert oder assoziierbar. Dazu ist die zweite Komponente des signalbildenden Systems mit einer partikulären Festphase assoziiert, welche zusätzlich mit dem Liganden kovalent oder über ein Bindungspaar A/B assoziiert ist oder über ein Bindungspaar A/B assoziierbar ist.

In einer bevorzugten Ausführungsform des erfindungsgemäßen Verfahrens zur Bestimmung eines Inhibitors eines proteolytisch aktiven Gerinnungsfaktors wird die Probe zusätzlich mit einem Fibrinaggregationshemmer vermischt. Fibrinaggregationshemmer sind Substanzen, die die Aggregation von Thrombin-induzierten Fibrinmonomeren verhindern. Auf diese Weise wird die Entstehung eines Fibringerinnsels in einer fibrinogenhaltigen Probe verhindert, welches ansonsten die Messung, beispielsweise durch Limitation der Diffusion und Quenching, negativ beeinträchtigen könnte. Bevorzugte Fibrinaggregationshemmer sind synthetische Peptide, wie z.B. ein Peptid der Sequenz Glycin, Prolin, Arginin, Prolin (kommerziell erhältlich als Pefabloc^{®} FG, Pentapharm, Schweiz). Weitere bevorzugte Peptide, die als Fibrinaggregationshemmer verwendet werden können, insbesondere das bevorzugte Peptid der Sequenz Glycin, Prolin, Arginin, Prolin, Alanin, sind in EP-A2-456 152 beschrieben.

In einer bevorzugten Ausführungsform des erfindungsgemäßen Verfahren zur Bestimmung eines direkten Inhibitors eines proteolytisch aktiven Gerinnungsfaktors wird dem Reaktionsansatz zusätzlich ein Polyamin wie Polybrene® (Hexadimethrinbromid), Spermin oder Polylysin zugegeben. Polyamine inhibieren die indirekte Inhibition des Gerinnungsfaktors durch Heparine. Auf diese Weise kann mit dem erfindungsgemäßen Verfahren zwischen Heparin-induzierter und direkter Inhibition unterschieden werden.

Nachdem der Reaktionsansatz, der die Probe, eine definierte Menge eines proteolytisch aktiven Gerinnungsfaktors, mindestens einen Liganden, der an das aktive Zentrum des proteolytisch aktiven Gerinnungsfaktors bindet, aber nicht von diesem an einer Peptidbindung gespalten wird, und eine erste und zweite Komponente eines signalbildenden Systems enthält, bereit gestellt ist, wird das Reaktionsgemisch für eine begrenzte Zeit inkubiert, um eine ausreichende Assoziation von proteolytisch aktivem Gerinnungsfaktor, Antikoagulanz und Ligand und gegebenenfalls von der ersten signalgebenden Komponente mit dem proteolytisch aktiven Gerinnungsfaktor und/oder von der zweiten signalgebenden Komponente mit dem Liganden zu gewährleisten. Der Begriff "ausreichend" ist so zu verstehen, dass das Verfahren als Ganzes eine quantitative Bestimmung Bestimmung eines Inhibitors eines proteolytisch aktiven Gerinnungsfaktors ermöglicht. Die optimale Inkubationsdauer eines bestimmten Testaufbaus kann experimentell ermittelt werden. Die Bereitstellung des Reaktionsansatzes kann in mehreren Einzelschritten mit unterschiedlicher Reihenfolge und unterschiedlichen Inkubationszeiten erfolgen.

Das Signal bzw. die Veränderung des Signals mit der Zeit, welches im Reaktionsgemisch entsteht, korreliert mit der Aktivität oder Menge des Inhibitors des proteolytisch aktiven Gerinnungsfaktors. Über diese Korrelation kann mit Hilfe des Signals bzw. der zeitlichen Änderung des Signals die Aktivität oder Menge eines Inhibitors des proteolytisch aktiven Gerinnungsfaktors ermittelt werden. Hierfür wird vorzugsweise eine Kalibration mit einem Standard durchgeführt. Der Standard besitzt eine definierte Aktivität oder Menge des Inhibitors des proteolytisch aktiven Gerinnungsfaktors.

Ein weiterer Gegenstand der vorliegenden Erfindung sind Testkits zur Durchführung der verschiedenen Ausführungsformen des erfindungsgemäßen Verfahrens.

Ein erstes Testkit enthält folgende separate Komponenten:
i. ein erstes Reagenz, das eine definierte Menge eines proteolytisch aktiven Gerinnungsfaktors enthält;
ii. ein zweites Reagenz, das mindestens einen Liganden, welcher an das aktive Zentrum des proteolytisch aktiven Gerinnungsfaktors bindet, aber nicht von diesem an einer Peptidbindung gespalten wird, enthält;
iii. ein drittes Reagenz, das eine erste Komponente eines signalbildenden Systems enthält, welche mit dem proteolytisch aktiven Gerinnungsfaktor aus dem ersten Reagenz assoziierbar ist; und
iv. ein viertes Reagenz, das eine zweite Komponente eines signalbildenden Systems enthält, welche mit dem Liganden aus dem zweiten Reagenz assoziierbar ist.

Bei der Verwendung eines solchen Testkits wird jede Komponente einzeln zu der Probe bzw. dem Reaktionsansatz gegeben, und die Assoziation des proteolytisch aktiven Gerinnungsfaktors mit der ersten Komponente des signalbildenden Systems und die Assoziation des Liganden mit der zweiten Komponente des signalbildenden Systems finden während der Inkubation im Reaktionsansatz statt. Dies hat den Vorteil, dass das dritte und vierte Reagenz jeweils ein universell einsetzbares Nachweisreagenz sein kann, sofern die Assoziation des proteolytisch aktiven Gerinnungsfaktors bzw. des Liganden über herkömmliche Bindungspaare A/B bzw. X/Y erfolgt, wie z.B. über Streptavidin/Biotin, Avidin/Biotin, Flag-Tag/anti-Flag-Tag-Antikörper oder ähnliches.

Ein anderes Testkit enthält folgende separate Komponenten:
i. ein erstes Reagenz, das eine definierte Menge eines proteolytisch aktiven Gerinnungsfaktors enthält, wobei der proteolytisch aktive Gerinnungsfaktor mit einer ersten Komponente eines signalbildenden Systems assoziiert ist;
ii. ein zweites Reagenz, das mindestens einen Liganden, welcher an das aktive Zentrum des proteolytisch aktiven Gerinnungsfaktors bindet, aber nicht von diesem an einer Peptidbindung gespalten wird, enthält; und
iii. ein drittes Reagenz, das eine zweite Komponente des signalbildenden Systems enthält, welche mit dem Liganden aus dem zweiten Reagenz assoziierbar ist.

Bei der Verwendung eines solchen Testkits findet lediglich die Assoziation des Liganden mit der zweiten Komponente des signalbildenden Systems während der Inkubation im Reaktionsansatz statt; der proteolytisch aktive Gerinnungsfaktors ist bereits mit der ersten Komponente des signalbildenden Systems assoziiert.

Ein noch anderes Testkit enthält folgende separate Komponenten:
i. ein erstes Reagenz, das eine definierte Menge eines proteolytisch aktiven Gerinnungsfaktors enthält;
ii. ein zweites Reagenz, das eine erste Komponente eines signalbildenden Systems enthält, welche mit dem proteolytisch aktiven Gerinnungsfaktor aus dem ersten Reagenz assoziierbar ist; und
iii. ein drittes Reagenz, das mindestens einen Liganden, an das aktive Zentrum des proteolytisch aktiven Gerinnungsfaktors bindet, aber nicht von diesem an einer Peptidbindung gespalten wird, enthält, und wobei der Ligand mit einer zweiten Komponente des signalbildenden Systems assoziiert ist.

Bei der Verwendung eines solchen Testkits findet lediglich die Assoziation des proteolytisch aktiven Gerinnungsfaktors mit der ersten Komponente des signalbildenden Systems während der Inkubation im Reaktionsansatz statt; der Ligand ist bereits mit der ersten Komponente des signalbildenden Systems assoziiert.

Ein noch anderes Testkit enthält folgende separate Komponenten:
i. ein erstes Reagenz, das eine definierte Menge eines proteolytisch aktiven Gerinnungsfaktors enthält, wobei der proteolytisch aktive Gerinnungsfaktor mit einer ersten Komponente eines signalbildenden Systems assoziiert ist; und
ii. ein zweites Reagenz, das mindestens einen Liganden enthält, welcher an das aktive Zentrum des proteolytisch aktiven Gerinnungsfaktors bindet, aber nicht von diesem an einer Peptidbindung gespalten wird, und wobei der Ligand mit einer zweiten Komponente des signalbildenden Systems assoziiert ist.

Bei der Verwendung eines solchen Testkits sind sowohl der proteolytisch aktive Gerinnungsfaktor als auch der Ligand bereits mit den jeweiligen Komponenten des signalbildenden Systems assoziiert.

Der Vorteil von Testkits, bei denen der proteolytisch aktive Gerinnungsfaktor und/oder der Ligand bereits mit der jeweiligen signalgebenden Komponente assoziiert sind, besteht unter anderem darin, dass die Anzahl der zu verwendenden Reagenzien minimiert wird, wodurch Pipettierschritte vermieden werden und das Risiko von Pipettierfehlern sinkt.

### Abkürzungsverzeichnis

- Ac: Acyl-
- Agm: Agmantin
- Ala: Alanin
- ANBA: 5-Amino-2-Nitro-Benzoesäure
- Arg: Arginin
- B-f-P-R-H: Biotinyl-Ttds--D-Phe-Pro-Arg-H
- Boc: Tertiärbutylcarbonate
- B-r-P-R-H: Biotinyl-Ttds--D-Arg-Pro-Arg-H
- Dpa: ß,ß-Dipenylalanin
- f: D-Phenylalanin
- Gly: Glycin
- Gpa: Guanidinophenylalanin
- His: Histidin
- IPA: Isoproplyamid
- Ki: Bindungkonstante
- Me: Methyl-
- Ph: Phenyl-
- Phe: Phenylalanin
- pNA: para-Nitroanilin
- Pro: Prolin
- r: D-Arginin
- R: Korrelationskoeffizient
- Tos: Tosyl-
- Ttds: 4,7,10-trioxa-1,13-tridecandiaminosuccinsäure
- Z-D-Leu: Carbobenzoxy-D-Leucin

### FIGURENBESCHREIBUNG

**Figur 1**
   Schematische Darstellung eines beispielhaften Testaufbaus für ein erfindungsgemäßes Verfahren zur Bestimmung von Thrombininhibitoren mittels LOCI-Technologie. Proteolytisch aktives Thrombin ist an eine partikuläre Festphase gebunden, die ferner mit einem chemilumineszierenden Agens beschichtet ist (Chemibead, CB). Die zweite Komponente des signalbildenden Systems besteht aus einer partikulären Festphase, die mit einem Photosensitizer und ferner mit Streptavidin beschichtet ist (Sensibead, SB).Ein Ligand, welcher an das aktive Zentrum von Thrombin bindet, aber nicht von diesem gespalten wird und welcher eine Biotin-Markierung aufweist, die an das Streptavidin des Sensibeads bindet, bringt die beiden Komponenten des signalbildenden Systems in räumliche Nähe. Die Anregung des Photosensitizers mit Licht verursacht die Entstehung von kurzlebigem Singulettsauerstoff, welcher das chemilumineszierende Agens zu aktivieren vermag, wodurch ein Lumineszenzsignal emittiert wird. Je mehr Thrombininhibitor (Antikoagulanz) in einer Probe enthalten ist, der mit dem nicht spaltbaren, biotinylierten Liganden um die Bindung an das Thrombin konkurriert, desto weniger Lumineszenzsignal wird erzeugt.
**Figur 2**
   Strukturformel des aminoterminalen Biotinyl-4,7,10-Trioxa-13-Tridecanaminamin Succinsäure (Biotinyl-Ttds) Linkers des Thrombin-spezifischen Liganden Biotinyl-Ttds-D-Phe-Pro-Arg-H und des Faktor Xa-spezifischen Liganden Biotinyl-Ttds-D-Arg-Gly-Arg-H.
**Figur 3**
   Bestimmung der Thrombin-Bindungskonstante von Biotinyl-Ttds-D-Phe-Pro-Arg-H (B-f-P-R-H). Darstellung der Abhängigkeit des Kehrwerts der Reaktionsgeschwindigkeit von der Ligandenkonzentration bei chromogenen Substratkonzentrationen von 4, 2 und 0,5 mM in einem Thrombintest gemäß Beispiel 2.
**Figur 4**
   Bestimmung der Thrombin-Bindungskonstante von Biotinyl-Ttds-D-Phe-Pro-Arg-H (B-f-P-R-H). Vergrößerter Ausschnitt aus Figur 3.
**Figur 5**
   Bestimmung der Thrombin-Bindungskonstante von Biotinyl-Ttds-D-Arg-Gly-Arg-H (B-r-G-R-H). Darstellung der Abhängigkeit des Kehrwerts der Reaktionsgeschwindigkeit von der Ligandenkonzentration bei chromogenen Substratkonzentrationen von 4, 2 und 0,5 mM in einem Thrombintest gemäß Beispiel 2.
**Figur 6**
   Bestimmung der F Xa-Bindungskonstante von Biotinyl-Ttds-D-Phe-Pro-Arg-H (B-f-P-R-H). Darstellung der Abhängigkeit des Kehrwerts der Reaktionsgeschwindigkeit von der Ligandenkonzentration bei chromogenen Substratkonzentrationen von 4, 2 und 0,5 mM in einem F Xa-Test gemäß Beispiel 3.
**Figur 7**
   Bestimmung der F Xa-Bindungskonstante von Biotinyl-Ttds-D-Arg-Gly-Arg-H (B-r-G-R-H).Darstellung der Abhängigkeit des Kehrwerts der Reaktionsgeschwindigkeit von der Ligandenkonzentration bei chromogenen Substratkonzentrationen von 4, 2 und 0,5 mM in einem F Xa-Test gemäß Beispiel 3.
**Figur 8**
   Chemilumineszenzsignal-Entstehung in Standard Humanplasma mit verschiedenen Konzentrationen der Thrombininhibitoren Fragmin, Liquemin, Argatroban, Melagatran und Refludan (Hirudin), wobei der Ligand Biotinyl-Ttds-D-Phe-Pro-Arg-H (B-f-P-R-H) in Verbindung mit Thrombin-beschichteten Chemibeads und Streptavidin-beschichteten Sensibeads eingesetzt wurde.
**Figur 9**
   Chemilumineszenzsignal-Entstehung in Standard Humanplasma mit verschiedenen Konzentrationen der FXa-Inhibitoren Fragmin, Liquemin und Rivaroxaban und der Thrombininhibitoren Refludan (Hirudin) und Argatroban, wobei der Ligand Biotinyl-Ttds-D-Arg-Gly-Arg-H (B-r-G-R-H) in Verbindung mit anti-FXa-Antikörper-beschichteten Chemibeads, FXa und Streptavidin-beschichteten Sensibeads eingesetzt wurde.

Die folgenden Beispiele dienen der Veranschaulichung der vorliegenden Erfindung und sind nicht als Einschränkung zu verstehen.

### Beispiel 1: Synthese von Thrombin- und Faktor Xa-Biotinyl-Ttds-Peptidaldehyd-Liganden

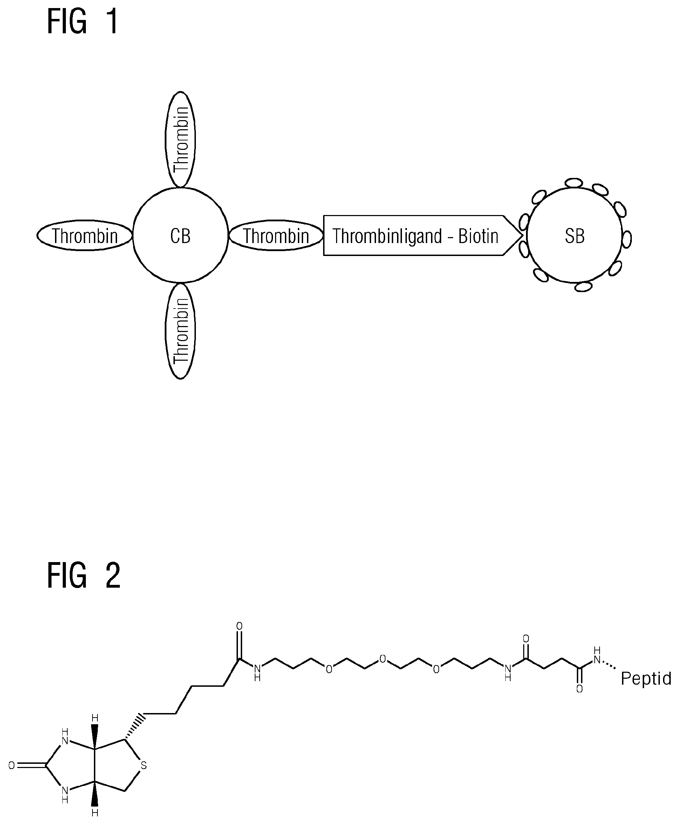

Die Peptidaldehyde -D-Phe-Pro-Arg-H (siehe Claeson, G., Blood Coagulation and Fibrinolysis 5, 1994, Seite 417) und -D-Arg-Gly-Arg-H (vergleiche Claeson, G., Blood Coagulation and Fibrinolysis 5, 1994, Seite 426) wurden auf einer Festphase synthetisiert, mit einem Ttds-Spacer (Ttds = 4,7,10-Trioxa-1,13-tridecandiamino Succinsäure (Bartos, A. et al., 2009, Biopolymers 92(2), 110-115) verlängert und mit Biotin versehen. Die Verbindung Biotinyl-Ttds-D-Phe-Pro-Arg-H hat ein Molekuargewicht von 930,15 g/mol und wird im folgenden kurz mit B-f-P-R-H bezeichnet. Die Verbindung Biotinyl-Ttds-D-Arg-Gly-Arg-H hat ein Molekulargewicht von 899,10 g/mol und wird im folgenden kurz mit B-r-G-R-H bezeichnet. Die Abspaltung der Peptidaldehyde von der Festphase erfolgte mit Trifluoressigsäure. Die Verbindungen wurden lyophilisiert bei -20 °C gelagert. Die Struktur des Biotinlinkers ist in Fig. 2 dargestellt

### Beispiel 2: Bestimmung der Thrombin-Bindungskonstanten der Peptidaldehyd-Liganden

Die kinetischen Daten der Peptidaldehyd-Liganden wurden chromogenen Testformaten durch Messen der Geschwindigkeit der Hydrolyse von chromogenen Peptidsubstraten, welche mit den Peptidaldehyd-Liganden um die Bindung an das aktive Zentrum des jeweiligen Enzyms konkurrieren, bei verschiedenen Substratkonzentrationen und Peptidaldehyd-Liganden-Konzentrationen bestimmt. Die Bindungskonstante (Ki) wurde nach einem bekannten Verfahren bestimmt (Dixon, M., 1953, Biochem J, 55, 170-171).

Die Bestimmung der Thrombin-Bindungskonstante wurde mit den Reagenzien des Hirudin Activity Tests von Siemens Healthcare Diagnostics durchgeführt. Der Hirudin Activity Test besteht einem lyophilisierten Thrombinreagenz bestehend aus Rinderthrombin, einem Heparininhibitor und Aprotinin, einem lyophilisierten chromogenen Substratreagenz mit einer Konzentration von 4 mmol/L tos-Gly-Pro-Arg-ANBA-IPA (tosylglycyl-L-propyl-arginyl-5-amino-2-nitrobenzoylisopropylamid) nach Rekonstitution. Das Thrombinreagenz wurde in Pufferlösung (Trishydroxymethylaminomethan, NaCl, pH 8,2) rekonstituiert. Von dem Substratreagenz wurden Verdünnungen mit deionisiertem Wasser hergestellt. Die gemäß Beispiel 1 hergestellten Peptidaldehyd-Liganden wurden in Wasser gelöst und als Probe eingesetzt. Der B-r-G-R-H-Ligand wurde in einem Konzentrationsbereich von 26,1 bis 0,815 µM eingesetzt. Der B-f-P-R-H-Ligand wurde in einem Konzentrationsbereich von 26,1 bis 0 µM eingesetzt. Die Durchführung der Teste erfolgte automatisch in einem BCS® XP Gerinnungsautomaten (Siemens Healthcare Diagnostics, Marburg, Deutschland).

Ein Reaktionsansatz wurde wie folgt zusammengemischt:
30 µl Probe (Peptidaldehyd-Ligand B-r-G-R-H bzw. B-f-P-R-H),
150 µl Thrombinreagenz,
50 µl chromogenes Substratreagenz (tos-Gly-Pro-Arg-ABNA-IPA).

Anhand der Zunahme der optischen Dichte des Reaktionsansatzes bei einer Wellenlänge von 405 nm wurde die Reaktionsgeschwindigkeit in mE/min bestimmt. Die optische Dichte der Reaktionsansätze wurde dazu in Zeitintervallen von 15 bis 40 Sekunden für den B-f-P-R-H-Peptidaldehyd-Liganden bzw. 5-16 Sekunden für das B-r-G-R-H-Peptidaldehyd-Liganden bestimmt.

Reversibel und irreversibel bindende Liganden können durch ihre Reaktionskinetik unterschieden werden. Trägt man 1/v, wobei v die Reaktionsgeschwindigkeit ist, gegen die Ligandenkonzentration (i) bei konstanter Konzentration (S) eines spaltbaren Substrates auf, so erhält man eine Gerade. Bei Verwendung von zwei oder mehreren unterschiedlichen Substratkonzentrationen (S₁, S₂... S_{N}) schneiden sich die Geraden in einem Punkt. Der Wert der Abszissenachse dieses Punktes entspricht -Kᵢ, wobei Kᵢ der Bindungskonstante entspricht, siehe z.B. Klebe, G., Wirkstoffdesign, 2. Auflage, Spektrum Akademischer Verlag, Heidelberg, 2009, Seiten 52-62 und Dixon, M., 1953, Biochem J, 55, 170-171). Bei einer irreversiblen Hemmung treffen sich die Kurven an einem Punkt auf der Abszisse, der wiederum -Kᵢ entspricht.

Tabelle 2 zeigt die im Reaktionsansatz eingesetzten Konzentrationen des Peptidaldehyd-Liganden B-f-P-R-H sowie die Kehrwerte der mit den verschiedenen Konzentrationen des chromogenen Substrats erhaltenen Reaktionsgeschwindigkeiten.

**Tabelle 2**

| | **1/Reaktionsgeschwindigkeit [min/E]** | | |
|---|---|---|---|
| **C_{B-f-P-R-H} [µM]** | **C_{Substrat} = 4 mM** | **C_{Substrat} = 2 mM** | **C_{Substrat} = 0,5 mM** |
| 26,08696 | 14,58931 | 28,40843 | 121,67418 |
| 16,30435 | 9,30221 | 18,08682 | 74,54798 |
| 13,04348 | 7,61108 | 14,42763 | 59,11941 |
| 9,78261 | 5,84267 | 11,17905 | 43,90039 |
| 6,52174 | 4,08943 | 7,85565 | 32,28213 |
| 5,21739 | 3,25123 | 6,16548 | 24,75736 |
| 3,26087 | 2,13002 | 4,02645 | 15,77139 |
| 2,60870 | 1,77535 | 3,36951 | 13,19230 |
| 1,63043 | 1,18258 | 2,16372 | 8,49129 |
| 1,30435 | 0,99739 | 1,79279 | 7,17521 |
| 0,81522 | 0,72743 | 1,16912 | 4,77479 |
| 0,65217 | 0,67366 | 1,02929 | 4,16189 |
| 0,40761 | 0,53337 | 0,70084 | 2,64461 |
| 0,32609 | 0,50125 | 0,66133 | 2,38165 |
| 0,20380 | 0,42975 | 0,50607 | 1,63785 |
| 0,16304 | 0,43955 | 0,52101 | 1,71097 |
| 0,08152 | 0,41427 | 0,45943 | 1,56479 |
| 0,04076 | 0,39301 | 0,42471 | 1,74741 |
| 0,02038 | 0,38457 | 0,41376 | 1,86941 |
| 0 | 0,38254 | 0,41286 | 1,93034 |

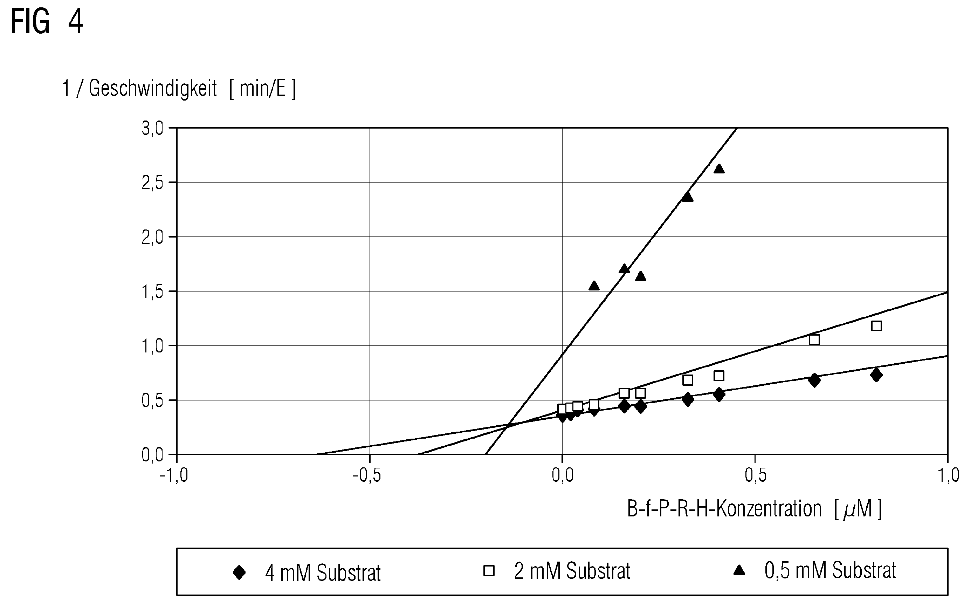

Es wurden die Regressiongeraden im linearen Bereich der Graphen 1/Reaktionsgeschwindigkeit gegen die Ligandenkonzentration (**C**_{B-f-P-R-H}) bei den verschiedenen Substratkonzentrationen aufgetragen (Figuren 3 und 4). Für C_{Substrat}=0,5 mM lagen die Konzentrationen von 0 bis 0,04076 µM nicht im linearen Bereich und wurden daher ausgeschlossen. Aus den Schnittpunkten der Regressionsgeraden wurden drei Kᵢ-Werte berechnet und daraus dann ein mittlerer Kᵢ-Wert und die Schnittpunkte der Graphen berechnet. Aus den so erhaltenen Kᵢ-Werten wurde der mittlere Kᵢ-Wert berechnet (Tabelle 3). Da die Schnittpunkte der Regressionsgeraden nicht auf der Abszisse liegen, handelt es sich um eine reversible Bindung des Peptidaldehyds mit Thrombin. Die mittlere Thrombin-Bindungskonstante K_{iM} des Peptidaldehyd-Liganden B-f-P-R-H beträgt 0,131 µmol (1,31 × 10⁻⁷ Mol) . Da die Bindungskonstanten der relevanten Medikamente (therapeutisch wirksame und geeignete Thrombininhibitoren) größer sind, ist die Verbindung entsprechend dem beschriebenen Verfahren, unter Verwendung von Thrombin als aktivem Gerinnungsenzym, geeignet zum Nachweis von hochaffinen Thrombininhibitoren.

**Tabelle 3**

| **Thrombin-Bindungskonstante (Kᵢ) von B-f-P-R-H** | | | | |
|---|---|---|---|---|
| **C_{Substrat}** (mM) | Steigung (min/E µM) | Achsenabschnitt (min/E) | R² | Kᵢ (µM) |
| 4 | 0,5500 | 0,3497 | 0,9997 | K_{i1&2}=0,106 |
| 2 | 1,0812 | 0,04059 | 0,9997 | K_{i1&3}=0,141 |
| 0,5 | 4,5718 | 0,9175 | 0,9994 | K_{i2&3}=0,147 |
| Mittlerer Ki: | | | | K_{iM}=0,131 |

Tabelle 4 zeigt die im Reaktionsansatz eingesetzten Konzentrationen des Peptidaldehyd-Liganden B-r-G-R-H sowie die Kehrwerte der mit den verschiedenen Konzentrationen des chromogenen Substrats erhaltenen Reaktionsgeschwindigkeiten.

**Tabelle 4**

| | **1/Reaktionsgeschwindigkeit [min/E]** | | |
|---|---|---|---|
| **C_{B-r-G-R-H} [µM]** | **C_{Substrat} = 4 mM** | **C_{Substrat} = 2 mM** | **C_{Substrat}= 0,5 mM** |
| 26,08696 | 0,39497 | 0,42847 | 0,69034 |
| 16,30435 | 0,38645 | 0,41405 | 0,62639 |
| 13,04348 | 0,38853 | 0,40320 | 0,60905 |
| 9,78261 | 0,38047 | 0,41056 | 0,58021 |
| 6,52174 | 0,38410 | 0,40077 | 0,56372 |
| 5,21739 | 0,37614 | 0,39456 | 0,54078 |
| 3,26087 | 0,38074 | 0,39679 | 0,53851 |
| 2,60870 | 0,37303 | 0,38756 | 0,52827 |
| 1,63043 | 0,37478 | 0,38938 | 0,52245 |
| 1,30435 | 0,37579 | 0,39024 | 0,52112 |
| 0,81522 | 0,37556 | 0,38842 | 0,51597 |

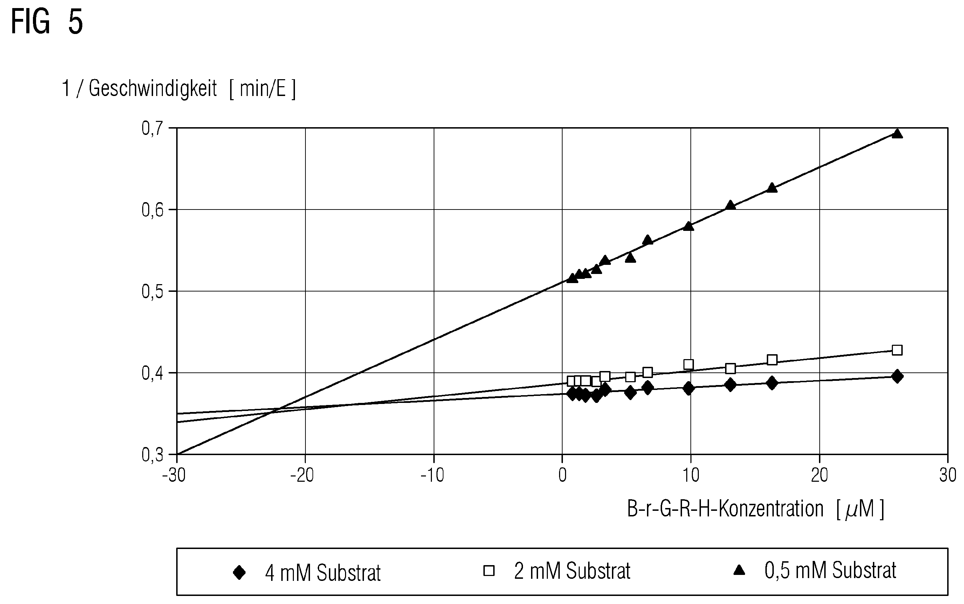

Es wurden die Regressiongeraden im linearen Bereich der Graphen 1/Reaktionsgeschwindigkeit gegen die Ligandenkonzentration (**C**_{B-r-G-R-H}) bei den verschiedenen Substratkonzentrationen aufgetragen (Figur 5). Aus den Schnittpunkten der Regressionsgeraden wurden drei Kᵢ-Werte berechnet und daraus dann ein mittlerer Kᵢ-Wert und die Schnittpunkte der Graphen berechnet. Aus den so erhaltenen Kᵢ-Werten wurde der mittlere Kᵢ-Wert berechnet (Tabelle 5). Da die Schnittpunkte der Regressionsgeraden nicht auf der Abszisse liegen, handelt es sich um eine reversible Bindung des Peptidaldehyds mit Thrombin. Die mittlere Thrombin-Bindungskonstante K_{iM} des Peptidaldehyd-Liganden B-r-G-R-H beträgt 20,9 µmol (2,09 × 10⁻⁵ Mol). Der Ligand ist daher geeignet entsprechend dem beschriebenen Verfahren, unter Verwendung von Thrombin als aktivem Gerinnungsenzym, auch schwach affine Thrombininhibitoren zu bestimmen.

**Tabelle 5**

| **Thrombin-Bindungskonstante (Kᵢ) von B-r-G-R-H** | | | | |
|---|---|---|---|---|
| **C_{Substrat}** (mM) | Steigung (min/E µM) | Achsenabschnitt (min/E) | R² | Kᵢ (µM) |
| 4 | 0,000805 | 0,3746 | 0,8520 | K_{i1&2}=17,4 |
| 2 | 0,001572 | 0,3880 | 0,9246 | K_{i1&3}=22,3 |
| 0,5 | 0,006964 | 0,5122 | 0,9544 | K_{i2&3}=23,0 |
| Mittlerer Ki: | | | | K_{iM}=20,9 |

### Beispiel 3: Bestimmung der F Xa-Bindungskonstanten der Peptidaldehyd-Liganden

Die Bestimmung der F Xa-Bindungskonstante wurde mit den Reagenzien des Berichrom® Heparin Tests von Siemens Healthcare Diagnostics durchgeführt. Der Berichrom® Heparin Test besteht aus:
- einem F Xa-Reagenz bestehend aus einer lyophilisierten Plasmafraktion, die Faktor Xa enthält, und Additiven wie Tris, NaCl, EDTA und Konservierungsmittel,
- einem chromogenen Substratreagenz (Z-D-Leu-Gly-Arg-ANBA-methyl-Amid),
- einem Verdünnungsreagenz zur Rekonstitution, und
- einem Dextransulfatreagenz bestehend aus lyophilisiertem Dextransulfat.

Nach Rekonstitution in 10 ml Verdünnungsreagenz ist die Konzentration des Dextransulfats bei 0,02 g/l. Das F Xa-Reagenz wird mit dem rekonstituierten Dextransulfatreagenz rekonstituiert. Das Substratreagenz enthält nach Rekonstitution mit 2 ml deionisiertem Wasser 4 mmol/L Z-D-Leu-Gly-Arg-ANBA-methyl-Amid. Von dem Substratreagenz wurden Verdünnungen mit deionisiertem Wasser hergestellt. Die gemäß Beispiel 1 hergestellten Peptidaldehyd-Liganden wurden in Wasser gelöst und als Probe eingesetzt. Die Durchführung der Teste erfolgte automatisch in einem BCS® XP Gerinnungsautomaten (Siemens Healthcare Diagnostics, Marburg, Deutschland).

Ein Reaktionsansatz wurde wie folgt zusammengemischt:
20 µl Wasser,
15 µl Probe (Peptidaldehyd-Ligand B-r-G-R-H bzw. B-f-P-R-H),
15 µl Wasser,
150 µl F Xa-Reagenz,
30 µl Substratreagenz (Z-D-Leu-Gly-Arg-ANBA-methyl-Amid).

Anhand der Zunahme der optischen Dichte des Reaktionsansatzes bei einer Wellenlänge von 405 nm wurde die Reaktionsgeschwindigkeit in mE/min bestimmt. Die optische Dichte der Reaktionsansätze wurde dazu in einem Zeitintervall von 10 bis 70 Sekunden bestimmt.

Tabelle 6 zeigt die im Reaktionsansatz eingesetzten Konzentrationen des Peptidaldehyd-Liganden B-f-P-R-H sowie die Kehrwerte der mit den verschiedenen Konzentrationen des chromogenen Substrats erhaltenen Reaktionsgeschwindigkeiten.

**Tabelle 6**

| | **1/Reaktionsgeschwindigkeit [min/E]** | | |
|---|---|---|---|
| **C_{B-f-P-R-H} [µM]** | **C_{Substrat} = 4 mM** | **C_{Substrat} = 2 mM** | **Csubstrat = 0,5 mM** |
| 13,04348 | 1,35879 | 2,49937 | 9,73911 |
| 8,15217 | 1,17310 | 2,14879 | 8,37740 |
| 6,52174 | 1,10558 | 1,97908 | 7,61262 |
| 4,89130 | 1,02567 | 1,83999 | 7,08399 |
| 3,26087 | 0,94477 | 1,71517 | 6,66273 |
| 2,60870 | 0,93172 | 1,67166 | 6,44104 |
| 1,63043 | 0,90212 | 1,59083 | 6,26347 |
| 1,30435 | 0,87349 | 1,56215 | 5,95893 |
| 0,81522 | 0,84935 | 1,52113 | 5,86778 |
| 0,65217 | 0,85137 | 1,50973 | 5,78490 |
| 0,40761 | 0,83380 | 1,48916 | 5,69390 |
| 0,32609 | 0,83198 | 1,48552 | 5,68996 |
| 0,20380 | 0,82383 | 1,47333 | 5,61861 |
| 0,16304 | 0,83329 | 1,48487 | 5,67486 |
| 0,10190 | 0,81565 | 1,46315 | 5,61945 |
| 0,08152 | 0,82180 | 1,46678 | 5,57154 |
| 0,04076 | 0,81738 | 1,45442 | 5,56930 |
| 0,02038 | 0,81918 | 1,45743 | 5,56461 |
| 0,01019 | 0,82168 | 1,45734 | 5,61679 |
| 0 | 0,83042 | 1,47267 | 5,68282 |

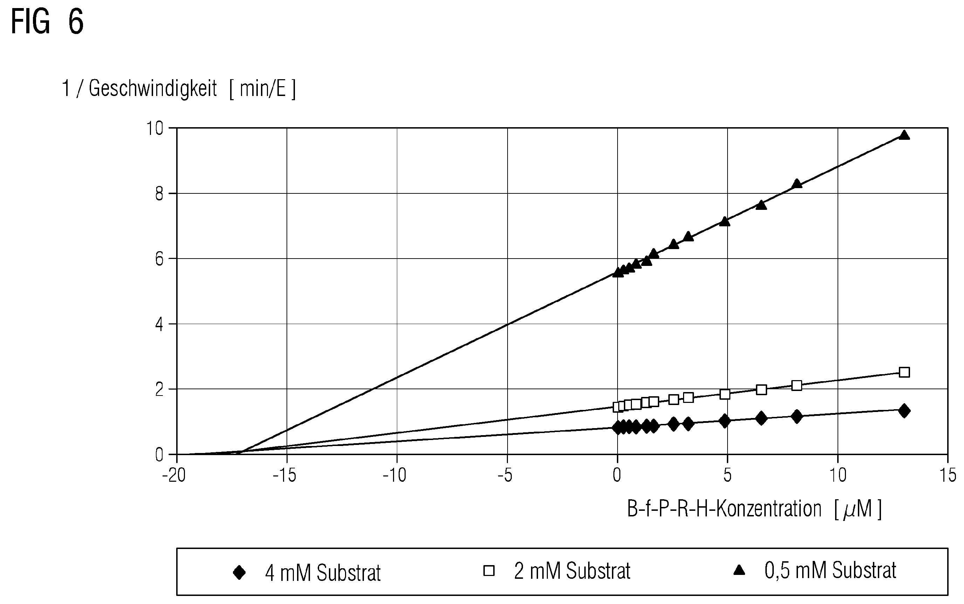

Es wurden die Regressionsgeraden im linearen Bereich der Graphen 1/Reaktionsgeschwindigkeit gegen die Ligandenkonzentration (**C**_{B-f-P-R-H}) bei den verschiedenen Substratkonzentrationen aufgetragen (Figur 6). Aus den Schnittpunkten der Regressionsgeraden wurden drei Kᵢ-Werte berechnet und daraus dann ein mittlerer Kᵢ-Wert und die Schnittpunkte der Graphen berechnet. Aus den so erhaltenen Kᵢ-Werten wurde der mittlere Kᵢ-Wert berechnet (Tabelle 7) Da die Schnittpunkte der Regressionsgeraden nicht auf der Abszisse liegen, handelt es sich um eine reversible Bindung des Peptidaldehyds mit F Xa. Die mittlere F Xa - Bindungskonstante K_{iM} des Peptidaldehyd-Liganden B-f-P-R-H beträgt 16,9 µmol (1,69 × 10⁻⁵ Mol). Der Ligand ist daher geeignet entsprechend dem beschriebenen Verfahren, unter Verwendung von F Xa als aktivem Gerinnungsenzym, schwach affine F Xa-Inhibitoren zu bestimmen.

**Tabelle 7**

| **F Xa-Bindungskonstante (Kᵢ) von B-f-P-R-H** | | | | |
|---|---|---|---|---|
| **C_{Substrat}** (mM) | Steigung (min/E µM) | Achsenabschnitt (min/E) | R² | Kᵢ (µM) |
| 4 | 0,0421 | 0,8202 | 0,9975 | K_{i1&2}=16,6 |
| 2 | 0,0806 | 1,4588 | 0,9987 | K_{i1&3}=17, |
| 0,5 | 0,3216 | 5,5934 | 0,9965 | K_{i2&3}=17,2 |
| Mittelerer Kᵢ: | | | | K_{iM}=16,9 |

Tabelle 8 zeigt die im Reaktionsansatz eingesetzten Konzentrationen des Peptidaldehyd-Liganden B-r-G-R-H sowie die Kehrwerte der mit den verschiedenen chromogenen Substratkonzentrationen erhaltenen Reaktionsgeschwindigkeiten.

**Tabelle 8**

| | **1**/**Reaktionsgeschwindigkeit [min/E]** | | |
|---|---|---|---|
| **C_{B-r-G-R-H} [µM]** | **C_{Substrat}= 4 mM** | **C_{Substrat} = 2 mM** | **C_{Substrat} = 0,5 mM** |
| 13,04348 | 8,46963 | 19,61291 | 98,11308 |
| 8,15217 | 5,66889 | 12,99196 | 62,32312 |
| 6,52174 | 4,81865 | 10,44658 | 52,69400 |
| 4,89130 | 3,74536 | 8,24448 | 37,79438 |
| 3,26087 | 2,72840 | 5,52006 | 26,41568 |
| 2,60870 | 2,27442 | 4,62654 | 20,14413 |
| 1,63043 | 1,67882 | 3,14700 | 13,97126 |
| 1,30435 | 1,47587 | 2,80172 | 12,04614 |
| 0,81522 | 1,17709 | 2,11479 | 8,40260 |
| 0,65217 | 1,09950 | 1,97479 | 7,75440 |
| 0,40761 | 0,92591 | 1,65890 | 6,40799 |
| 0,32609 | 0,90610 | 1,61219 | 6,12959 |
| 0,20380 | 0,82623 | 1,45744 | 5,52072 |

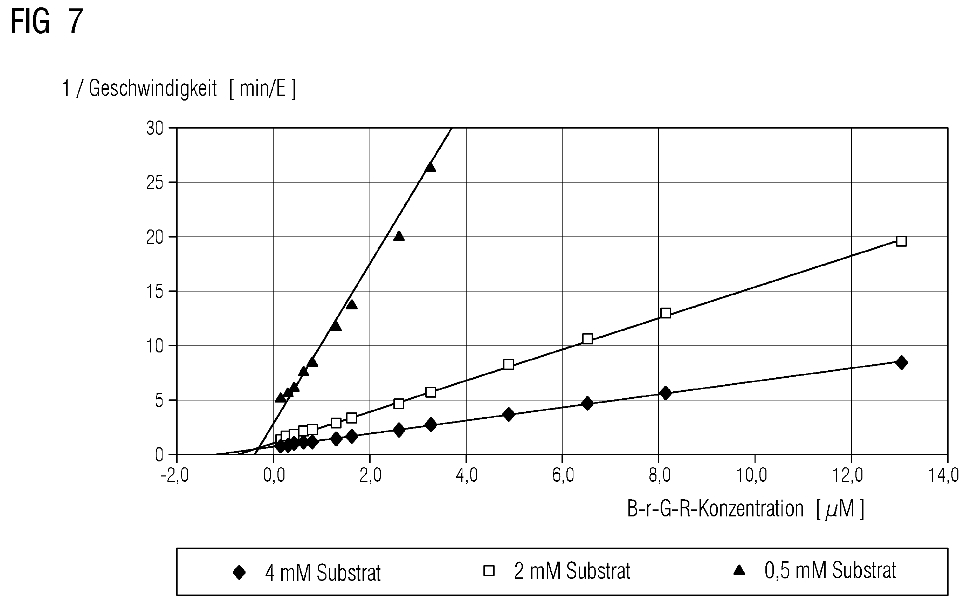

Es wurden die Regressiongeraden im linearen Bereich der Graphen 1/Reaktionsgeschwindigkeit gegen die Ligandenkonzentration (**C**_{B-r-G-R-H}) bei den verschiedenen Substratkonzentrationen aufgetragen (Figur 7). Aus den Schnittpunkten der Regressionsgeraden wurden drei Kᵢ-Werte berechnet und daraus dann ein mittlerer Kᵢ-Wert und die Schnittpunkte der Graphen berechnet. Aus den so erhaltenen Kᵢ-Werten wurde der mittlere Kᵢ-Wert berechnet (Tabelle 9). Da die Schnittpunkte der Regressionsgeraden nicht auf der Abszisse liegen, handelt es sich um eine reversible Bindung des Peptidaldehyds mit F Xa. Die mittlere F Xa Bindungskonstante K_{iM} des Peptidaldehyd-Liganden B-r-G-R-H beträgt 0,332 µmol (3,32 × 10⁻⁷ Mol). Da die Bindungskonstanten der relevanten Medikamente (therapeutisch wirksame und geeignete F Xa-Inhibitoren) größer sind, ist die Verbindung entsprechend dem beschriebenen Verfahren, unter Verwendung von F Xa als aktivem Gerinnungsenzym, geeignet zum Nachweis von hochaffinen F Xa-Inhibitoren.

**Tabelle 9**

| **F Xa**-**Bindungskonstante (Kᵢ) von B**-**r**-**G**-**R**-**H** | | | | |
|---|---|---|---|---|
| **C_{Substrat}** (mM) | Steigung (min/E µM) | Achsenabschnitt (min/E) | R² | Kᵢ (µM) |
| 4 | 0,6037 | 0,7187 | 0,9992 | K_{i1&2}=0,358 |
| 2 | 1,4375 | 1,0169 | 0,9992 | K_{i1&3}=0,321 |
| 0,5 | 7,3110 | 2,8743 | 0,9987 | K_{i2&3}=0,316 |
| Mittelerer Kᵢ: | | | | K_{iM}=0,332 |

### Beispiel 4: Erfindungsgemäßes Verfahren zur Bestimmung von Thrombininhibitoren mittels LOCI-Reagenzien

Als Proben wurden verwendet Standard Humanplasma sowie Standard Humanplasma, dem steigende Konzentrationen direkter (Hirudin, Melagatran, Argatroban) bzw. indirekter (Liquemin, Fragmin) Thrombininhibitoren zugesetzt wurden.

Als signalbildendes System wurde das LOCI®-System verwendet bestehend aus einer Chemilumineszenzverbindung (2-(4-(N,N, di- tetradecyl)-anilino-3-phenyl thioxen) und einem Photosensitizer (bis-(trihexyl)-silicon-t-butyl-phthalocyanine). Sowohl die Chemilumineszenzverbindung als auch der Photosensitizer sind an Latexpartikel gekoppelt. Im folgenden werden auch die Begriffe "Chemibeads" für die mit der Chemilumineszenzverbindung beschichteten Partikel und "Sensibeads" für die mit dem Photosensitizer beschichteten Partikel verwendet. Die hier verwendete LOCI-Technologie beruht darauf, dass die Latexpartikel-gekoppelte Chemilumineszenzverbindung (Chemibeads) und der Latexpartikel-gekoppelte Photosensibilisator (Sensibeads) durch Bindung an einen Analyten in eine räumliche Nähe zueinander gebracht werden, so dass Singulett-Sauerstoff, der vom Photosensibilisator erzeugt wird, die Chemilumineszenzverbindung anregen kann.

Die hier verwendeten Chemibeads wurden zusätzlich mit Rinderthrombin beschichtet. Dazu wurde Rinderthrombin kovalent mit den Latexpartikeln verbunden. Die hier verwendeten Sensibeads wurden zusätzlich mit Streptavidin beschichtet.

Als Ligand wurde das biotinylierte Peptidaldehyd Biotinyl-Ttds-D-Phe-Pro-Arg-H **(B-f-P-R-H)** verwendet.

In Abwesenheit eines Thrombininhibitors bindet der Ligand über seinen Biotinrest an die Streptavidin-beschichteten Sensibeads und über seinen Peptidanteil an die Thrombin-beschichteten Chemibeads und ein Chemilumineszenzsignal wird erzeugt (siehe auch Figur 1). In Anwesenheit eines Thrombininhibitors, der mit dem Liganden um die Bindung an die Thrombin-beschichteten Chemibeads konkurriert, aber nicht an die Sensibeads binden kann, verhält sich das Chemilumineszenzsignal umgekehrt proportional zur Thrombininhibitorkonzentration.

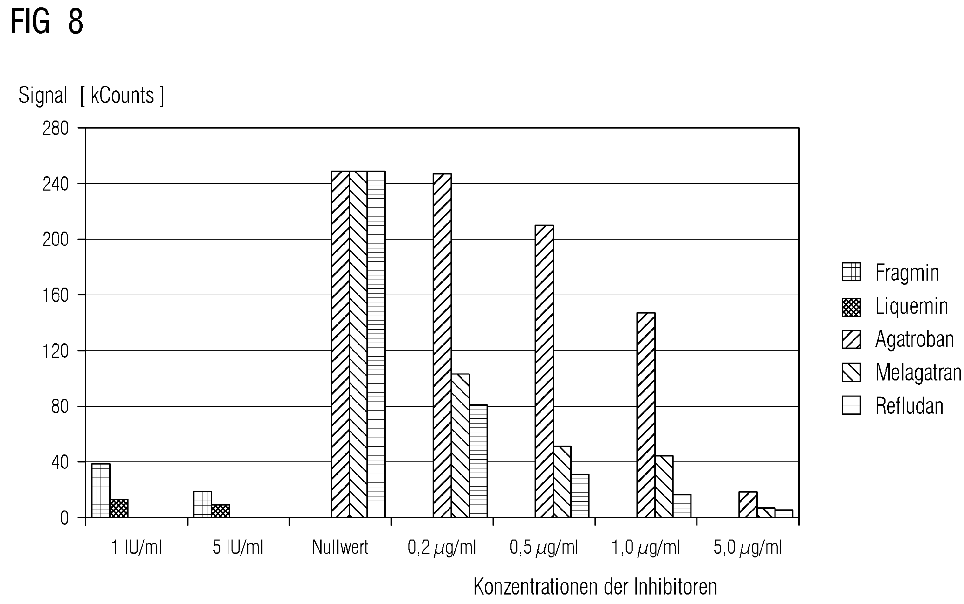

Es wurden jeweils 2 µL Probe, 10 µL des Fibrinpolymerisationsinhibitor-Peptids Glycin-Prolin-Argin-Prolin (10 mg/mL), 20 µL Chemibeads (400 µg/mL) und 10 µL des Liganden (1,25 µM) zusammengefügt und für 6 Minuten inkubiert. Nach Zugabe von 20 µL Sensibeads (600 µg/mL) und einer Inkubation von 10 Minuten wurde der Ansatz mit Wasser ad 250 µL aufgefüllt und das Chemilumineszenzsignal gemessen. Wie in Figur 8 gezeigt, verringert sich das Chemilumineszenzsignal in Anhängigkeit von der Art und Konzentration der zudosierten direkten Thrombininhibitoren. Auch zudosierte Heparine (Liquemin, Fragmin), die Thrombin indirekt hemmen, führen zu einer deutlichen Verringerung des Chemilumineszenzsignals.

### Beispiel 5: Erfindungsgemäßes Verfahren zur Bestimmung von Faktor Xa-Inhibitoren mittels LOCI-Reagenzien

Als Proben wurden verwendet Standard Humanplasma sowie Standard Humanplasma, dem steigende Konzentrationen direkter bzw. indirekter Thrombin- (Hirudin, Argatroban, Liquemin) oder Faktor Xa- (Rivaroxaban, Liquemin, Fragmin) Inhibitoren zugesetzt wurden.

Als signalbildendes System wurde das LOCI-System gemäß Beispiel 4 verwendet.

Die hier verwendeten Chemibeads wurden zusätzlich mit einem kommerziell erhältlichen polyklonalen Antikörper gegen FXa beschichtet. Dazu wurde der Antikörper kovalent mit den Latexpartikeln in einem Kopplungsverhältnis von 10 mg Antikörper auf 50 ng Partikel verbunden. Die hier verwendeten Sensibeads wurden zusätzlich mit Streptavidin beschichtet.

Als Ligand wurde das biotinylierte Peptidaldehyd Biotinyl-Ttds-D-Arg-Gly-Arg-H (B-r-G-R-H) verwendet.

In Abwesenheit eines FXa-Inhibitors bindet der Ligand über seinen Biotinrest an die Streptavidin-beschichteten Sensibeads und über seinen Peptidanteil an den FXa, der selbst an die Antikörper-beschichteten Chemibeads bindet, und ein Chemilumineszenzsignal wird erzeugt (siehe auch Figur 1). In Anwesenheit eines FXa-Inhibitors, der mit dem Liganden um die Bindung an die FXa-beschichteten Chemibeads konkurriert, aber nicht an die Sensibeads binden kann, verhält sich das Chemilumineszenzsignal umgekehrt proportional zur FXa-Inhibitorkonzentration (z.B. Rivaroxaban). In Anwesenheit eines Thrombinhibitors (z.B. Argatroban oder Hirudin) wird dagegen das Signal nicht reduziert, da der Thrombininhibitor den Liganden nicht verdrängen kann.

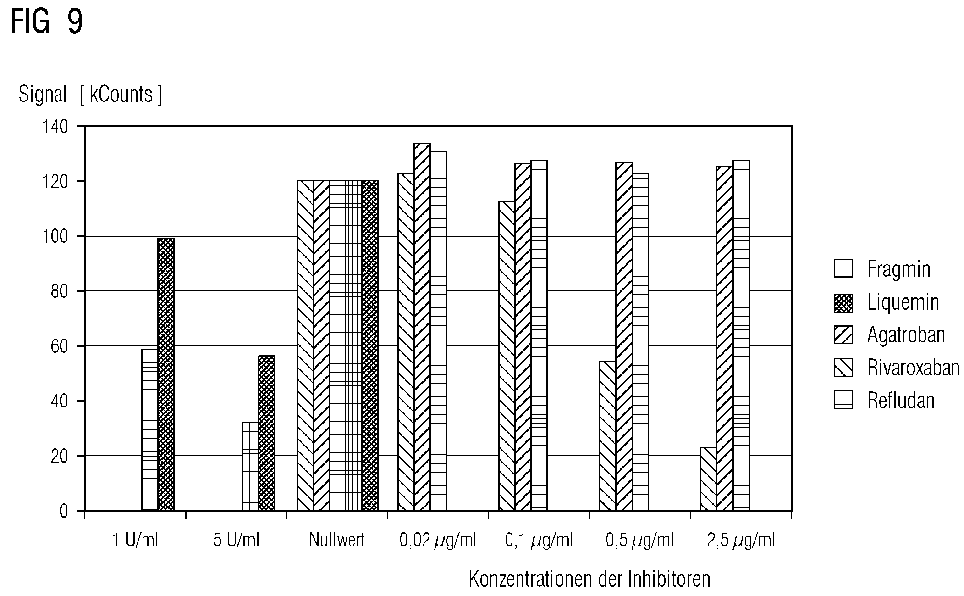

Es wurden 10 µL eines Reagenzes enthaltend humanen FXa (ca. 1.000 IU/L) und Fibrinpolymerisationsinhibitor-Peptid Glycin-Prolin-Argin-Prolin (10 mg/mL) mit 2 µL Probe, 20 µL Chemibeads (400 µg/mL) und 10 µL des Liganden (6 µM) vermischt und für 5 Minuten inkubiert. Nach Zugabe von 60 µL Sensibeads (200 µg/mL) und einer Inkubation von 10 Minuten wurde der Ansatz mit Wasser ad 260 µL aufgefüllt und das Chemilumineszenzsignal gemessen. Wie in Figur 9 gezeigt, verringert sich das Chemilumineszenzsignal in Abhängigkeit von der Art und Konzentration der zudosierten direkten FXa-und Thrombininhibitoren. Auch zudosierte Heparine (Liquemin, Fragmin), die FXa indirekt hemmen, führen zu einer deutlichen Verringerung des Chemilumineszenzsignals.

## Patentansprüche

1. Verfahren zur Bestimmung eines Inhibitors eines proteolytisch aktiven Gerinnungsfaktors in einer Probe, umfassend die Schritte:
a) Bereitstellen und Inkubieren eines Reaktionsansatzes, enthaltend
i. ein Aliquot der Probe,
ii. eine definierte Menge eines proteolytisch aktiven Gerinnungsfaktors,
iii. mindestens einen Liganden, welcher an das aktive Zentrum des proteolytisch aktiven Gerinnungsfaktors bindet, aber nicht von diesem an einer Peptidbindung gespalten wird,
iv. eine erste und eine zweite Komponente eines signalbildenden Systems, welche so zusammenwirken, dass ein detektierbares Signal entsteht, wenn die erste und die zweite Komponente des signalbildenden Systems in räumliche Nähe zueinander gebracht werden und wobei der proteolytisch aktive Gerinnungsfaktor mit der ersten Komponente des signalbildenden Systems assoziiert ist oder während der Inkubation assoziiert wird und wobei der Ligand mit der zweiten Komponente des signalbildenden Systems assoziiert ist oder während der Inkubation assoziiert wird;
b) Messen des Signals des signalbildenden Systems, wobei sich die Signalhöhe umgekehrt proportional zur Inhibitorkonzentration in der Probe verhält.

2. Verfahren nach Anspruch 1, wobei der Ligand, welcher an das aktive Zentrum des proteolytisch aktiven Gerinnungsfaktors bindet, eine Bindungsaffinität von Ki = von 10⁻¹³ bis 10⁻⁴ M für den proteolytisch aktiven Gerinnungsfaktor aufweist.

3. Verfahren nach einem der Ansprüche 1 oder 2, wobei die erste und zweite Komponente des signalbildenden Systems jeweils eine partikuläre Festphase umfassen, und wobei die Agglutination der partikulären Festphasen im Reaktionsansatz gemessen wird.

4. Verfahren nach einem der Ansprüche 1 oder 2, wobei die erste Komponente des signalbildenden System ein chemilumineszierendes Agens ist und die zweite Komponente des signalbildenden Systems ein Photosensitizer ist oder umgekehrt und wobei die Chemilumineszenz im Reaktionsansatz gemessen wird.

5. Verfahren nach Anspruch 4, wobei das chemilumineszierende Agens mit einer ersten partikulären Festphase assoziiert ist und/oder der Photosensitizer mit einer zweiten partikulären Festphase assoziiert ist.

6. Verfahren nach einem der Ansprüche 1 bis 5, wobei der Ligand einen ersten Bindungspartner A eines ersten Bindungspaares A/B aufweist und wobei die zweite Komponente des signalbildenden Systems den zweiten Bindungspartner B des ersten Bindungspaares A/B aufweist und wobei der Ligand durch Bindung der Bindungspartner A und B mit der zweiten Komponente des signalbildenden Systems assoziiert ist oder während der Inkubation assoziiert wird.

7. Verfahren nach einem der Ansprüche 1 bis 6, wobei der Ligand ein Peptidderivat ist.

8. Verfahren nach einem der Ansprüche 1 bis 7, wobei der Ligand ein Peptidderivat ist, das eine carboxyterminale Gruppe aus der Gruppe Aldehyd, Keton, Trifluormethylketon, α-Ketokarbonsäure, α-Ketoamid, α-Ketoester, Ester, Boronsäure, Chlormethylketon und Sulfonylfluorid aufweist.

9. Verfahren nach Anspruch 6, wobei die Bindungspartner A und B so gewählt sind, dass sie ein Bindungspaar A/B aus der Gruppe FLAG-Tag/anti-FLAG-Tag-Antikörper, HIS-Tag/anti-HIS-Tag-Antikörper, Fluorescein/anti-Fluorescein-Antikörper, Biotin/Avidin und Biotin/Streptavidin bilden.

10. Verfahren nach einem der vorherigen Ansprüche, wobei der proteolytisch aktive Gerinnungsfaktor einen ersten Bindungspartner X eines zweiten Bindungspaares X/Y aufweist und wobei der zweite Bindungspartner Y des zweiten Bindungspaares X/Y mit der ersten Komponente des signalbildenden Systems assoziiert ist und wobei der proteolytisch aktive Gerinnungsfaktor durch Bindung der Bindungspartner X und Y mit der ersten Komponente des signalbildenden Systems assoziiert ist oder während der Inkubation assoziiert wird.

11. Verfahren nach einem der vorherigen Ansprüche zur Bestimmung eines Thrombininhibitors, wobei ein Reaktionsansatz bereit gestellt und inkubiert wird, der eine definierte Menge des proteolytisch aktiven Gerinnungsfaktors Thrombin enthält.

12. Verfahren nach Anspruch 11, wobei ein Reaktionsansatz bereit gestellt und inkubiert wird, der das Peptid Biotinyl-Ttds-D-Phe-Pro-Arg-H enthält, welches reversibel an das aktive Zentrum von Thrombin bindet, aber nicht von diesem gespalten wird,.

13. Verfahren nach einem der Ansprüche 11 oder 12 zur Bestimmung eines Thrombininhibitors aus der Gruppe Heparin, Heparinderivate, Bivalirudin, Hirudin, Dabigatran, Argatroban, Melagatran, Ximelagatran Lepirudin, MCC-977, SSR-182289, TGN-255, TGN-167, ARC-183 und Odiparcil.

14. Verfahren nach einem der Ansprüche 1 bis 10 zur Bestimmung eines Faktor Xa-Inhibitors, wobei ein Reaktionsansatz bereit gestellt und inkubiert wird, der eine definierte Menge des proteolytisch aktiven Gerinnungsfaktors Faktor Xa enthält.

15. Verfahren nach Anspruch 14 zur Bestimmung eines Faktor Xa-inhibitors aus der Gruppe Heparin, Heparinderivate wie Fondaparinux, Rivaroxaban, Apixaban, Otamixaban, LY 517717, YM 153, DU-176b, DX-9065a und KFA-1982.

16. Verfahren nach einem der Ansprüche 14 oder 15, wobei ein Reaktionsansatz bereit gestellt und inkubiert wird, der das Peptid Biotinyl-Ttds-D-Arg-Gly-Arg-H enthält, welches reversibel an das aktive Zentrum von Faktor Xa bindet, aber nicht von diesem gespalten wird.

17. Testkit zur Durchführung eines Verfahrens gemäß einem der Ansprüche 1 bis 16, wobei das Testkit folgende separate Komponenten enthält:
i. ein erstes Reagenz, das eine definierte Menge eines proteolytisch aktiven Gerinnungsfaktors enthält;
ii. ein zweites Reagenz, das mindestens einen Liganden, welcher an das aktive Zentrum des proteolytisch aktiven Gerinnungsfaktors bindet, aber nicht von diesem an einer Peptidbindung gespalten wird, enthält;
iii. ein drittes Reagenz, das eine erste Komponente eines signalbildenden Systems enthält, welche mit dem proteolytisch aktiven Gerinnungsfaktor aus dem ersten Reagenz assoziierbar ist; und
iv. ein viertes Reagenz, das eine zweite Komponente eines signalbildenden Systems enthält, welche mit dem Liganden aus dem zweiten Reagenz assoziierbar ist;
wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

18. Testkit zur Durchführung eines Verfahrens gemäß einem der Ansprüche 1 bis 16, wobei das Testkit folgende separate Komponenten enthält:
i. ein erstes Reagenz, das eine definierte Menge eines proteolytisch aktiven Gerinnungsfaktors enthält, wobei der proteolytisch aktive Gerinnungsfaktor mit einer ersten Komponente eines signalbildenden Systems assoziiert ist;
ii. ein zweites Reagenz, das mindestens einen Liganden, welcher an das aktive Zentrum des proteolytisch aktiven Gerinnungsfaktors bindet, aber nicht von diesem an einer Peptidbindung gespalten wird, enthält; und
iii. ein drittes Reagenz, das eine zweite Komponente des signalbildenden Systems enthält, welche mit dem Liganden aus dem zweiten Reagenz assoziierbar ist;
wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

19. Testkit zur Durchführung eines Verfahrens gemäß einem der Ansprüche 1 bis 16, wobei das Testkit folgende separate Komponenten enthält:
i. ein erstes Reagenz, das eine definierte Menge eines proteolytisch aktiven Gerinnungsfaktors enthält;
ii. ein zweites Reagenz, das eine erste Komponente eines signalbildenden Systems enthält, welche mit dem proteolytisch aktiven Gerinnungsfaktor aus dem ersten Reagenz assoziierbar ist; und
iii. ein drittes Reagenz, das mindestens einen Liganden, welcher an das aktive Zentrum des proteolytisch aktiven Gerinnungsfaktors bindet, aber nicht von diesem an einer Peptidbindung gespalten wird, enthält, und wobei der Ligand mit einer zweiten Komponente des signalbildenden Systems assoziiert ist;
wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

20. Testkit zur Durchführung eines Verfahrens gemäß einem der Ansprüche 1 bis 16, wobei das Testkit folgende separate Komponenten enthält:
i. ein erstes Reagenz, das eine definierte Menge eines proteolytisch aktiven Gerinnungsfaktors enthält, wobei der proteolytisch aktive Gerinnungsfaktor mit einer ersten Komponente eines signalbildenden Systems assoziiert ist; und
ii. ein zweites Reagenz, das mindestens einen Liganden enthält, welcher an das aktive Zentrum des proteolytisch aktiven Gerinnungsfaktors bindet, aber nicht von diesem an einer Peptidbindung gespalten wird, und wobei der Ligand mit einer zweiten Komponente des signalbildenden Systems assoziiert ist;
wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

## Claims

1. Method of determining an inhibitor of a proteolytically active coagulation factor in a sample, comprising the steps of:
a) providing and incubating a reaction mix comprising
i. an aliquot of the sample,
ii. a defined amount of a proteolytically active coagulation factor,
iii. at least one ligand which binds to the active site of the proteolytically active coagulation factor but is not cleaved by the latter at a peptide bond,
iv. a first and a second component of a signal-producing system which act together in such a way that a detectable signal is generated when said first and second components of said signal-producing system are brought into close proximity to each other, and wherein the proteolytically active coagulation factor is associated with the first component of the signal-producing system or will be associated therewith during incubation, and wherein the ligand is associated with the second component of the signal-producing system or will be associated therewith during incubation;
b) measuring the signal of the signal-producing system, wherein the amplitude of said signal is inversely proportional to the inhibitor concentration in the sample.

2. Method according to Claim 1, wherein the ligand which binds to the active site of the proteolytically active coagulation factor has a binding affinity of Ki = from 10⁻¹³ to 10⁻⁴ M to the proteolytically active coagulation factor.

3. Method according to either of Claims 1 and 2, wherein the first and second components of the signal-producing system each comprise a particulate solid phase, and wherein agglutination of the particulate solid phases in the reaction mix is measured.

4. Method according to either of Claims 1 and 2, wherein the first component of the signal-producing system is a chemiluminescent agent and the second component of the signal-producing system is a photosensitizer or vice versa, and wherein chemiluminescence in the reaction mix is measured.

5. Method according to Claim 4, wherein the chemiluminescent agent is associated with a first particulate solid phase and/or the photosensitizer is associated with a second particulate solid phase.

6. Method according to any of Claims 1 to 5, wherein the ligand has a first binding partner A of a first binding pair A/B, and wherein the second component of the signal-producing system has the second binding partner B of the first binding pair A/B, and wherein the ligand, due to binding of the binding partners A and B, is associated with the second component of the signal-producing system or will be associated therewith during incubation.

7. Method according to any of Claims 1 to 6, wherein the ligand is a peptide derivative.

8. Method according to any of Claims 1 to 7, wherein the ligand is a peptide derivative having a carboxy terminal group from the group consisting of aldehyde, ketone, trifluoromethyl ketone, α-ketocarboxylic acid, α-ketoamide, α-keto ester, ester, boronic acid, chloromethyl ketone, and sulfonyl fluoride.

9. Method according to Claim 6, wherein the binding partners A and B are chosen such that they form a binding pair A/B from the group consisting of FLAG Tag/anti-FLAG tag antibody, HIS tag/anti-HIS tag antibody, fluorescein/anti-fluorescein antibody, biotin/avidin, and biotin/streptavidin.

10. Method according to any of the preceding claims, wherein the proteolytically active coagulation factor has a first binding partner X of a second binding pair X/Y, and wherein the second binding partner Y of the second binding pair X/Y is associated with the first component of the signal-producing system, and wherein the proteolytically active coagulation factor, due to binding of the binding partners X and Y, is associated with the first component of the signal-producing system or will be associated therewith during incubation.

11. Method according to any of the preceding claims for determining a thrombin inhibitor, wherein a reaction mix is provided and incubated which comprises a defined amount of the proteolytically active coagulation factor thrombin.

12. Method according to Claim 11, wherein a reaction mix is provided and incubated which comprises the peptide biotinyl-Ttds-D-Phe-Pro-Arg-H which binds reversibly to the active site of thrombin but is not cleaved thereby.

13. Method according to either of Claims 11 and 12 for determining a thrombin inhibitor from the group consisting of heparin, heparin derivatives, bivalirudin, hirudin, dabigatran, argatroban, melagatran, ximelagatran, lepirudin, MCC-977, SSR-182289, TGN-255, TGN-167, ARC-183 and odiparcil.

14. Method according to any of Claims 1 to 10 for determining a factor Xa inhibitor, wherein a reaction mix is provided and incubated which comprises a defined amount of the proteolytically active coagulation factor factor Xa.

15. Method according to Claim 14 for determining a factor Xa inhibitor from the group consisting of heparin, heparin derivatives such as fondaparinux, rivaroxaban, apixaban, otamixaban, LY 517717, YM 153, DU-176b, DX-9065a and KFA-1982.

16. Method according to either of Claims 14 and 15, wherein a reaction mix is provided and incubated which comprises the peptide biotinyl-Ttds-D-Arg-Gly-Arg-H which binds reversibly to the active site of factor Xa but is not cleaved thereby.

17. Test kit for carrying out a method according to any of Claims 1 to 16, said test kit comprising the following separate components:
i. a first reagent comprising a defined amount of a proteolytically active coagulation factor;
ii. a second reagent comprising at least one ligand which binds to the active site of the proteolytically active coagulation factor but is not cleaved by the latter at a peptide bond;
iii. a third reagent comprising a first component of a signal-producing system, which component is associable with the proteolytically active coagulation factor from the first reagent; and
iv. a fourth reagent comprising a second component of a signal-producing system, which component is associable with the ligand from the second reagent;
wherein the first and second components of the signal-producing system act together in such a way that a detectable signal is generated when said first and second components of said signal-producing system are brought into close proximity to each other.

18. Test kit for carrying out a method according to any of Claims 1 to 16, said test kit comprising the following separate components:
i. a first reagent comprising a defined amount of a proteolytically active coagulation factor, wherein the proteolytically active coagulation factor is associated with a first component of a signal-producing system;
ii. a second reagent comprising at least one ligand which binds to the active site of the proteolytically active coagulation factor but is not cleaved by the latter at a peptide bond; and
iii. a third reagent comprising a second component of the signal-producing system, which component is associable with the ligand from the second reagent;
wherein the first and second components of the signal-producing system act together in such a way that a detectable signal is generated when said first and second components of said signal-producing system are brought into close proximity to each other.

19. Test kit for carrying out a method according to any of Claims 1 to 16, said test kit comprising the following separate components:
i. a first reagent comprising a defined amount of a proteolytically active coagulation factor;
ii. a second reagent comprising a first component of a signal-producing system, which component is associable with the proteolytically active coagulation factor from the first reagent; and
iii. a third reagent comprising at least one ligand which binds to the active site of the proteolytically active coagulation factor but is not cleaved by the latter at a peptide bond, and wherein the ligand is associated with a second component of the signal-producing system;
wherein the first and second components of the signal-producing system act together in such a way that a detectable signal is generated when said first and second components of said signal-producing system are brought into close proximity to each other.

20. Test kit for carrying out a method according to any of Claims 1 to 16, said test kit comprising the following separate components:
i. a first reagent comprising a defined amount of a proteolytically active coagulation factor, wherein the proteolytically active coagulation factor is associated with a first component of a signal-producing system; and
ii. a second reagent comprising at least one ligand which binds to the active site of the proteolytically active coagulation factor but is not cleaved by the latter at a peptide bond, and wherein the ligand is associated with a second component of the signal-producing system;
wherein the first and second components of the signal-producing system act together in such a way that a detectable signal is generated when said first and second components of said signal-producing system are brought into close proximity to each other.

## Revendications

1. Procédé de détermination d'un inhibiteur d'un facteur de coagulation actif protéolytiquement dans un échantillon, comprenant les stades :
a) On prépare et on fait incuber une masse réactionnelle contenant
i. un aliquot de l'échantillon,
ii. une quantité définie d'un facteur de coagulation actif protéolytiquement,
iii. au moins un ligand, qui se fixe au centre actif du facteur de coagulation actif protéolytiquement, mais qui n'est pas scindé de celui-ci sur une liaison peptide,
iv. un premier et un deuxième constituants d'un système formant un signal, qui coopèrent de manière à créer un signal détectable si le premier et le deuxième constituants du système formant un signal sont mis à proximité l'un de l'autre dans l'espace, et dans lequel le facteur de coagulation actif protéolytiquement est associé au premier constituant du système formant un signal ou est associé pendant l'incubation et dans lequel le ligand est associé au deuxième constituant du système formant un signal ou est associé pendant l'incubation.
b) On mesure le signal du système formant un signal, l'amplitude du signal étant inversement proportionnelle à la concentration de l'inhibiteur dans l'échantillon.

2. Procédé suivant la revendication 1, dans lequel le ligand, qui se fixe au centre actif du facteur de coagulation actif protéolytiquement, a une affinité de liaison de Ki = de 10⁻¹³ à 10⁻⁴ M pour le facteur de coagulation actif protéolytiquement.

3. Procédé suivant l'une des revendications 1 ou 2, dans lequel le premier et le deuxième constituants du système formant un signal comprennent respectivement une phase solide en particules, et dans lequel on mesure l'agglutination des phases solides en particules dans la masse réactionnelle.

4. Procédé suivant l'une des revendications 1 ou 2, dans lequel le premier constituant du système formant un signal est un agent chémioluminescent et le deuxième constituant du système formant un signal est un photosensibilisateur ou inversement, et dans lequel on mesure la chémioluminescence dans la masse réactionnelle.

5. Procédé suivant la revendication 4, dans lequel l'agent chémioluminescent est associé à une première phase solide en particules et/ou le photosensibilisateur est associé à une deuxième phase solide en particules.

6. Procédé suivant l'une des revendications 1 à 5, dans lequel le ligand a un premier partenaire A de liaison d'une première paire A/B de liaison et le deuxième constituant du système formant un signal a le deuxième partenaire B de liaison de la première paire A/B de liaison, et dans lequel le ligand est associé au deuxième constituant du système formant un signal par une liaison des partenaires A et B de liaison ou est associé pendant l'incubation.

7. Procédé suivant l'une des revendications 1 à 6, dans lequel le ligand est un dérivé de peptide.

8. Procédé suivant l'une des revendications 1 à 7, dans lequel le ligand est un dérivé de peptide, qui a un groupe à terminaison carboxy choisi dans le groupe aldéhyde, cétone, trifluorométhylcétone, acide α-cétocarboxylique, α-cétoamide, α-cétoester, ester, acide borique, chlorométhylcétone et fluorure de sulfonyle.

9. Procédé suivant la revendication 6, dans lequel on choisit les partenaires A et B de liaison de manière à former une paire A/B de liaison composée du groupe FLAG-Tag-anticorps/anti-FLAG-Tag, HIS-Tag/anticorps-HIS-Tag, fluorescéine/anticorps-antifluorescéine, biotine/avidine et biotine/streptavidine.

10. Procédé suivant l'une des revendications précédente, dans lequel le facteur de coagulation actif protéolytiquement a un premier partenaire X de liaison d'une deuxième paire X/Y de liaison, et dans lequel le deuxième partenaire Y de liaison de la deuxième paire X/Y de liaison est associé au premier constituant du système formant un signal, et dans lequel le facteur de coagulation actif protéolytiquement est associé au premier constituant du système formant un signal par une liaison des partenaires X et Y de liaison ou est associé pendant l'incubation.

11. Procédé suivant l'une des revendications précédentes, pour la détermination d'un inhibiteur de la trombine, dans lequel on prépare et on fait incuber une masse réactionnelle qui contient une quantité définie de la trombine comme facteur de coagulation actif protéolytiquement.

12. Procédé suivant la revendication 11, dans lequel on prépare et fait incuber une masse réactionnelle qui contient le peptide biotinyle-Ttds-D-Phe-Pro-Arg-H, lequel se fixe réversiblement sur le centre actif de la trombine, mais ne se scinde pas de celui-ci.

13. Procédé suivant l'une des revendications 11 ou 12, de détermination d'un inhibiteur de la trombine choisi dans le groupe de l'héparine, d'un dérivé d'héparine, de la bivalirudine, de l'hirudine, du dabigatran, de l'argatroban, du mélagatran, du ximélagatran, de la lépirudine, du MCC-977, du SSR-182289, du TGN-255, du TGN-167, de l'ARC-183 et de l'odiparcile.

14. Procédé suivant l'une des revendications 1 à 10, de détermination d'un inhibiteur du facteur Xa, dans lequel on prépare et on fait incuber une masse réactionnelle qui contient une quantité dérivée du facteur Xa comme facteur de coagulation actif protéolytiquement.

15. Procédé suivant la revendication 14, de détermination d'un inhibiteur du facteur Xa choisi dans le groupe de l'héparine, d'un dérivé de l'héparine, comme le fondaparinux, le rivaroxaban, l'apixaban, l'otamixaban, le LY 517717, le YM 153, le DU-176b, le DX-9065a et le KFA-1982.

16. Procédé suivant l'une des revendications 14 ou 15, dans lequel on prépare et fait incuber une masse réactionnelle qui contient le peptide biotinyle-Ttds-D-Arg-Gly-Arg-H qui se fixe réversiblement sur le centre actif du facteur Xa, mais ne se scinde pas de celui-ci.

17. Trousse de test pour effectuer un procédé suivant l'une des revendication 1 à 16, la trousse de test contenant les constituants séparés suivants :
i. un premier réactif, qui contient une quantité définie d'un facteur de coagulation actif protéolytiquement ;
ii. un deuxième réactif, qui contient au moins un ligand qui se fixe au centre actif du facteur de coagulation actif protéolytiquement, mais qui ne se scinde pas de celui-ci sur une liaison peptide ;
iii. un troisième réactif, qui contient un premier constituant d'un système formant un signal et qui peut être associé au facteur de coagulation actif protéolytiquement du premier réactif et
iv. un quatrième réactif, qui contient un constituant d'un système formant un signal et qui peut être associé au ligand du deuxième réactif ;
dans laquelle le premier et le deuxième constituants du système formant un signal coopèrent entre eux, de manière à créer un signal détectable si le premier et le deuxième constituants du système formant un signal sont mis l'un par rapport à l'autre à proximité dans l'espace.

18. Trousse de test pour effectuer un procédé suivant l'une des revendication 1 à 16, dans laquelle la trousse de test contient les constituants suivants séparés :
i. un premier réactif, qui contient une quantité définie d'un facteur de coagulation actif protéolytiquement, le facteur de coagulation actif protéolytiquement étant associé à un premier constituant d'un système formant un signal ;
ii.un deuxième réactif, qui contient un ligand qui se fixe au centre actif du facteur de coagulation actif protéolytiquement, mais qui ne se scinde pas de celui-ci sur une liaison peptide et
iii. un troisième réactif, qui contient un premier constituant d'un système formant un signal et qui peut être associé au ligand du deuxième réactif ;
dans laquelle le premier et le deuxième constituants du système formant un signal coopèrent entre eux, de manière à créer un signal détectable si le premier et le deuxième constituants du système formant un signal sont mis l'un par rapport à l'autre à proximité dans l'espace.

19. Trousse de test pour effectuer un procédé suivant l'une des revendication 1 à 16, dans laquelle la trousse de test contient des constituants suivants séparés :
i. un premier réactif, qui contient une quantité définie d'un facteur de coagulation actif protéolytiquement;
ii. un deuxième réactif, qui contient un premier constituant d'un système formant un signal et qui peut être associé au facteur de coagulation actif protéolytiquement du premier réactif et ;
iii. un troisième réactif, qui contient au moins un ligand qui se fixe au centre actif du facteur de coagulation actif protéolytiquement, mais qui ne se scinde pas de celui-ci sur une liaison peptide, et dans lequel le ligand est associé à un deuxième constituant du système formant un signal ;
dans laquelle le premier et le deuxième constituants du système formant un signal coopèrent entre eux, de manière à créer un signal détectable si le premier et le deuxième constituants du système formant un signal sont mis l'un par rapport à l'autre à proximité dans l'espace.

20. Trousse de test pour effectuer un procédé suivant l'une des revendication 1 à 16, dans laquelle la trousse de test contient les constituants suivants séparés :
i. un premier réactif, qui contient une quantité définie d'un facteur de coagulation actif protéolytiquement, le facteur de coagulation actif protéolytiquement étant associé à un premier constituant d'un système formant un signal et
ii.un deuxième réactif, qui contient au moins un ligand qui se fixe au centre actif du facteur de coagulation actif protéolytiquement, mais ne se scinde pas de celui-ci sur une liaison peptide, et dans lequel le ligand est associé à un deuxième constituant du système formant un signal ;
dans laquelle le premier et le deuxième constituants du système formant un signal coopèrent entre eux, de manière à créer un signal détectable si le premier et le deuxième constituants du système formant un signal sont mis l'un par rapport à l'autre à proximité dans l'espace.
